# EUROPÄISCHE PATENTSCHRIFT

(11) **EP 2 370 065 B1**
(45) Veröffentlichungstag und Bekanntmachung des Hinweises auf die Patenterteilung: **04.05.2016**
(21) Anmeldenummer: 09755847.2
(22) Anmeldetag: 19.11.2009
(51) Int. Cl.: A61K 9/28, A61K 31/4178, A61K 31/4184, A61K 31/4422

(54) **PHARMAZEUTISCHE DARREICHUNGSFORM ENTHALTEND NIFEDIPIN ODER NISOLDIPIN UND EINEN ANGIOTENSIN-II ANTAGONISTEN UND/ODER EIN DIURETIKUM**
PHARMACEUTICAL DOSAGE FORM COMPRISING NIFEDIPINE OR NISOLDIPINE AND AN ANGIOTENSIN-II ANTAGONIST AND/OR A DIURETIC
FORME GALÉNIQUE PHARMACEUTIQUE CONTENANT DE LA NIFÉDIPINE OU DE LA NISOLDIPINE ET UN ANTAGONISTE DE L'ANGIOTENSINE II ET/OU UN DIURÉTIQUE

(30) Priorität: 27.11.2008 DE 102008059206
(43) Veröffentlichungstag der Anmeldung: 05.10.2011
(73) Patentinhaber: Bayer Intellectual Property GmbH, 40789 Monheim (DE)
(72) Erfinder: KUHL, Alexander, 100020 Beijing (CN); BRENDEL, Erich, 42657 Solingen (DE); BRÖCKER, Frank, 58285 Gevelsberg (DE); FUNKE, Adrian, 14055 Berlin (DE); OHM, Andreas, 41468 Neuss (DE); KVESIC, Dennis, Edmonton, Alberta, T5E 6H2 (CA); VOLKMER, Thomas, 14195 Berlin (DE)
(74) Vertreter: BIP Patents
(86) Internationale Anmeldenummer: PCT/EP2009/008232
(87) Internationale Veröffentlichungsnummer: WO 2010/060564

(56) Entgegenhaltungen:
- WO-A1-03/097045
- WO-A1-2008/044862
- WO-A2-2007/003330
- DE-A1- 19 747 261

## Beschreibung

Die vorliegende Erfindung betrifft eine pharmazeutische Darreichungsform enthaltend eine Wirkstoffkombination von Nifedipin oder Nisoldipin und mindestens einem Angiotensin-II Antagonisten und/oder mindestens einem Diuretikum, dadurch gekennzeichnet, dass der Kern ein osmotisches Freisetzungssystemen ist, dadurch gekennzeichnet, dass die Hülle des osmotischen Freisetzungssystems aus Celluloseacetat oder einem Gemisch aus Celluloseacetat und Polyethylenglykol besteht, sowie weiterhin dadurch gekennzeichnet, dass sich Nifedipin oder Nisoldipin im Kern und der Angiotensin II Antagonist und/oder das Diuretikum in einem Mantelüberzug um den Kern befinden und dadurch gekennzeichnet, dass der Mantelüberzug mindestens ein Film bildendes Polymer geeignet für die schnelle Freisetzung von Wirkstoffen enthält, wobei das Film bildende Polymer partiell hydrolysierter Polyvinylalkohol ist und weiterhin dadurch gekennzeichnet, dass mindestens 85 % des Nifedipins oder Nisoldipins (bezogen auf die deklarierte Gesamtmenge des jeweiligen Wirkstoffes) über einen Zeitraum von 24 Stunden, 5 bis 17 % des Nifedipins oder Nisoldipins innerhalb von 4 Stunden und 43 bis 80 % des Nifedipins oder Nisoldipins innerhalb von 12 Stunden freigesetzt werden gemäß USP-Freisetzungsmethode mit Apparatur 2 (Paddle) und mindestens 60 % des Angiotensin-II Antagonisten und/oder des Diuretikums (bezogen auf die deklarierte Gesamtmenge des jeweiligen Wirkstoffes) innerhalb von 30 Minuten freigesetzt werden gemäß USP-Freisetzungsmethode mit Apparatur 2 (Paddle) bei 75 Umdrehungen pro Minute in 900 ml geeignetem Medium, sowie Verfahren zu ihrer Herstellung, ihre Anwendung als Arzneimittel, sowie ihre Verwendung zur Prophylaxe, Sekundärprophylaxe oder Behandlung von Erkrankungen.

Das primäre Ziel der pharmakologischen Therapie der Hypertonie besteht darin, den Blutdruck zu kontrollieren, um Folgeerkrankungen wie Herz-Kreislauf-Erkrankungen, zerebrovaskuläre Erkrankungen und Endorganschäden zu verhindern. Bei der Ersteinstellung von Bluthochdruckpatienten wird im Allgemeinen mit einer Monotherapie eines Bluthochdruckpraeparates begonnen (European Society of Hypertension Leitlinien 2007, Joint National Committee VII (JNC VII) Leitlinien, Japanese Society of Hypertension (JSH) Leitlinien). Es ist zu erwarten, dass beim Einsatz von Monotherapien mit Hypertoniepraeparaten eine Reihe von Patienten nicht den geforderten Zielblutdruck, wie in den internationalen Leitlinien beschrieben ist, erreichen wird. In den Vereinigten Staaten von Amerika werden ca 33% der Hypertonie Patienten, welche auf Monotherapie eingestellt sind, auf eine Zweitlinien-Therapie innerhalb des ersten Jahres der Therapie umgestellt.

Calciumantagonisten wie beispielsweise Nifedipin und Nisoldipin werden in der Therapie des Bluthochdruckes als bewährte Wirkstoffe erfolgreich eingesetzt. Die aufgeführten Beispiele sind dem Fachmann gut bekannt und in der einschlägigen Literatur beschrieben. Durch ihre direkte Wirkung auf die arteriellen Blutgefäße senken sie den Blutdruck bei einem großen Anteil der Patienten zuverlässig. Allerdings bewirken sie an der Niere durch bevorzugte Dilatation der Vasa afferentia einen Anstieg des Filtrationsdruckes. Dies kann bei vorgeschädigter Niere zu einer erhöhten Belastung des Filtrationsapparates führen und sich bei Patienten in einer Proteinurie bemerkbar machen. Dieser Effekt kann durch Hinzufügen einer therapeutisch wirksamen Dosis eines Angiotensin-II Antagonisten verhindert werden. Als Angiotensin-II Antagonisten eignen sich alle bekannten Angiotensin-II Antagonisten wie beispielsweise Azilsartan, Candesartan, Embursartan, Eprosartan, Irbesartan, Losartan, Telmisartan, Valsartan oder Olmesartan. Die aufgeführten Beispiele sind dem Fachmann gut bekannt und in der einschlägigen Literatur beschrieben. Da Angiotensin-II Antagonisten auch im Bereich des Vas efferens dilatatorisch wirksam sind, kann die zusätzliche Gabe von diesen Substanzen den unerwünschten Anstieg des Filtrationsdruckes verhindern.

Wie aus Hayashi K; Nagahama T, Oka K, Epstein M, Sarute T: Disparate effects of calcium antagonists on renal microcirculation. Hypertens Res 1996: 19: 31-36 bekannt, bewirkt die Kombination von Nifedipin oder Nisoldipin mit einem Angiotensin-II Antagonisten eine sehr gute Blutdrucksenkung verbunden mit einer geringen Belastung der Niere. Dies stellt einen erheblichen therapeutischen Fortschritt dar. Darüber hinaus können durch die Kombination auch andere Nebenwirkungen wie die bei Calciumantagonisten auftretenden peripheren Ödeme und die durch reflektorische Freisetzung von Noradrenalin bedingte Stimulierung des sympathischen Nervensystems verringert werden. Des weiteren zeigen jüngste Studienergebnisse (ACCOMPLISH; American Cardiolygy Congress Jamerson KA, et al. March 31, 2008; Chicago, IL.) einen Vorteil von festen Kombinationen nicht nur hinsichtlich der Blutdruckkontrollraten, sondern auch bei Verwendung eines Calciumantagonisten hinsichtlich der Senkung der cardiovaskularen Morbidität und Mortalität.

Bei Krankheiten, die über einen längeren Zeitraum behandelt werden müssen, oder zur längerfristigen Prophylaxe von Krankheiten, ist es wünschenswert, die Häufigkeit der Einnahme von Medikamenten so gering wie möglich zu halten. Dies ist nicht nur bequemer für den Patienten, sondern es erhöht auch die Behandlungssicherheit, indem es die Nachteile unregelmäßiger Einnahmen vermindert. Die gewünschte Reduktion der Einnahmefrequenz, beispielsweise von zweimal täglicher auf einmal tägliche Applikation, kann über eine Verlängerung der therapeutisch effektiven Plasmaspiegel durch modifizierte Wirkstofffreigabe aus den Darreichungsformen erreicht werden.

Nach Einnahme von Darreichungsformen mit modifizierter Wirkstofffreisetzung kann außerdem durch Glättung des Plasmaspiegelverlaufes (Minimierung des so genannten peak-trough-Verhältnisses), also durch Vermeidung von hohen Plasmawirkstoffkonzentrationen, die häufig nach Gabe schnellfreisetzender Arzneiformen zu beobachten sind, das Auftreten unerwünschter, mit den Konzentrationsspitzen korrelierten Nebenwirkungen vermindert werden.

Insbesondere für die Dauertherapie oder -prophylaxe und Sekundärprophylaxe von cardiovaskulären Erkrankungen ist es von Vorteil, die Wirkstoffe in einer Form zur Verfügung zu haben, die über eine modifizierte Wirkstofffreigabe zu einer Verringerung des peak-trough-Verhältnisses führt und eine einmal tägliche Applikation ermöglicht.

Bei der Formulierungsentwicklung sind weiterhin die physikalisch-chemischen und biologischen Eigenschaften der Wirkstoffe zu berücksichtigen, beispielsweise die relativ geringe Wasserlöslichkeit des Nifedipins (ca. 9 mg/l) und die Plasmahalbwertszeit von ca. 2 Stunden. Für die gewünschte einmal tägliche Applikation sind demnach spezielle galenische Formulierungen notwendig, die Nifedipin oder Nisoldipin unter Berücksichtigung seiner physikochemischen und biologischen Eigenschaften modifiziert freisetzen.

Die Angiotensin-II Antagonisten werden in Form ihrer Handelsprodukte sämtlich als schnellfreisetzende (immediate release (IR)) Formulierungen vermarktet, da sie trotz ihrer kurzen dominanten Plasmahalbwertszeit eine über 24 Stunden anhaltende Wirkung besitzen. Deshalb ist es wünschenswert, eine pharmazeutische Darreichungsform bereitzustellen, die mindestens einen Angiotensin-II Antagonisten und Nifedipin oder Nisoldipin enthält, wobei der Angiotensin-II Antagonist schnell und das Nifedipin oder Nisoldipin modifiziert freigesetzt wird.

Im Hinblick auf die biologischen Eigenschaften von Nifedipin oder Nisoldipin und den Angiotensin-II Antagonisten ist es entscheidend, dass beide Wirkstoffe aus den tiefen Darmabschnitten ohne signifikanten Bioverfügbarkeitsverlust resorbiert werden. Das ist nur bei ca. 30-50% aller Wirkstoffe der Fall und daher ist die entsprechende Auswahl der Kombinationswirkstoffe von entscheidender Bedeutung bei der Entwicklung eines IR-Retard-Kombinations-produktes.

Diuretika sind Arzneimittel, die zur Ausschwemmung von Wasser aus dem menschlichen oder tierischen Körper eingesetzt werden. Zum Teil werden auch vermehrt Salze ausgeschieden. Dies führt zu einer Verringerung des Plasmavolumens sowie des peripheren Widerstandes. Primär werden Diuretika zur Absenkung des Blutdrucks eingesetzt. Man unterscheidet verschiedene Typen von Diuretika. Carboanhydrasehemmstoffe (Acetazolamid): Blockade der Protonensekretion und Natriumbicarbonat-Rückresorption, überwiegend am proximalen Tubulus. Verwendung fast nur noch bei Glaukombehandlung in der Augenheilkunde. Schleifendiuretika (Furosemid, Torasemid, Bumetanid, Etacrynsäure, Piretanid): reversible Hemmung eines Na/2Cl/K-Carriersystems am dicken Teil der aufsteigenden Henle'sche Schleife. Kaliumsparende Diuretika (Amilorid, Triamteren): Blockade der Na-Kanäle am spätdistalen Tubulus und am Sammelrohr, Hemmung der Na-Rückresorption, dadurch verminderte K-Sekretion. Aldosteronantagonisten (Spironolacton, Kaliumcanreonat, Eplerenon): kompetitive Bindung am Aldosteronrezeptor, dadurch Hemmung der Na-Rückresorption und K-Sekretion, eingesetzt bei Aszites in Zusammenhang mit Leberzirrhose und als Zusatztherapeutikum bei chronischer Herzinsuffizienz. Thiaziddiuretika und andere Sulfonamid-Diuretika (Hydrochlorothiazid (=HCTZ), Chlorothiazid, Chlorthalidon, Xipamid, Indapamid, Mefrusid): reversible Hemmung des Na-Cl-Kotransports am frühdistalen Tubulus (luminal), Hemmung der Carboanhydrase, Verminderung der GFR, Hydrochlorothiazid sehr oft mit Antihypertonika kombiniert eingesetzt. Das Hinzufuegen eines Diuretikums wie beispielsweise HCTZ bei Mono-Therapie verstaerkt die blutdrucksenkende Wirkung der Kombination.

Kombinationen aus einem Diuretikum und Angiotensin-II Antagonisten sind dem Fachmann bekannt, beispielsweise aus EP 1 306 088 B (Candesartan und Furoseemid) aber auch folgende Fix Dosis Kombinationen zur Behandlung des Bluthochdrucks wie beispielsweise Hyzaar® (= Losartan Kalium plus HCTZ) von Merck, Co-Diovan® (= Valsartan plus HCTZ) von Novartis oder Boehringer s Micardis Plus® (= Telmisartan plus HCTZ).

Kombinationen aus einem Angiotensin-II Antagonisten und Calcium Kanalblockern einerseits oder Diuretika andererseits sind dem Fachmann bekannt, beispielsweise aus WO 92/10097. Explizit werden die Kombinationen aus Eprosartan und Nifedipin und Eprosartan und Hydrochlorthiazid beschrieben. Konkret offenbart werden schnellfreisetzende Hartgelatinekapseln und Tabletten.

Darreichungsformen, die die Wirkstoffe Nifedipin oder Nisoldipin in Kombination mit einem Angiotensin-II Antagonisten modifiziert/retardiert freisetzen, sowie ihre Herstellung sind beispielweise in WO 2007/003330 beschrieben. Bei diesen Formulierungen werden sowohl das Nifedipin als auch der Angiotensin-II Antagonist retardiert freigesetzt.

Die Diuretika weisen unterschiedlich lange Plasmahalbwertszeiten und Wirkdauern auf, werden aber überwiegend in Form ihrer Handelsprodukte als schnellfreisetzende (immediate release (IR)) Formulierungen zur einmal täglichen Applikation vermarktet. Deshalb ist es wünschenswert, eine pharmazeutische Darreichungsform bereitzustellen, die mindestens einen Angiotensin-II Antagonisten und / oder ein Diuretikum und Nifedipin oder Nisoldipin enthält, wobei der Angiotensin-II Antagonist und/ oder das Diuretikum schnell und das Nifedipin oder Nisoldipin modifiziert freigesetzt wird.

Für die Herstellung modifiziert freisetzender pharmazeutischer Darreichungsformen sind verschiedene Methoden bekannt, siehe beispielsweise B. Lippold in "Oral Controlled Release Products: Therapeutic and Biopharmaceutic Assessment" Hrsg. U. Gundert-Remy und H. Möller, Stuttgart, Wiss.Verl.-Ges., 1989, 39-57.

Darreichungsformen, die die Wirkstoffe Nifedipin oder Nisoldipin modifiziert/retardiert freisetzen, sowie ihre Herstellung sind beispielweise in EP 0 299 211, EP 0 386 440, EP 0 776 660 und WO 2003/080057 beschrieben.

Besonders geeignete Darreichungsformen, die die Wirkstoffe Nifedipin oder Nisoldipin modifiziert/retardiert freisetzen, basieren auf osmotischen Freisetzungssystemen. Hierbei werden Kerne, beispielsweise Kapseln oder Tabletten, vorzugsweise Tabletten, mit einer semipermeablen Membran umgeben, die mindestens eine Öffnung aufweist. Die wasserdurchlässige Membran ist für die Komponenten des Kerns undurchlässig, erlaubt aber den Eintritt von Wasser von außen über Osmose in das System. Das eingedrungene Wasser setzt dann über den entstehenden osmotischen Druck den Wirkstoff gelöst oder suspendiert aus der oder den Öffnung/en in der Membran frei. Die Gesamtwirkstofffreisetzung und die Freisetzungsrate können im Wesentlichen über die Dicke und Porosität der semipermeablen Membran, die Zusammensetzung des Kerns und die Anzahl und Größe der Öffnung/en gesteuert werden. Vorteile, Formulierungsaspekte, Anwendungsformen und Informationen zu Herstellverfahren sind u. a. in folgenden Publikationen beschrieben:
- Santus, G., Baker, R.W., "Osmotic drug delivery: a review of the patent literature", Journal of Controlled Release 35 (1995), 1-21
- Verma, R.K., Mishra, B., Garg, S., "Osmotically controlled oral drug delivery", Drug Development and Industrial Pharmacy 26 (7), 695-708 (2000)
- Verma, R.K., Krishna, D.M., Garg, S., "Formulation aspects in the development of osmotically controlled oral drug delivery systems", Journal of Controlled Release 79 (2002), 7-27
- Verma, R.K., Arora, S., Garg, S., "Osmotic pumps in drug delivery", Critical Reviews in Therapeutic Drug Carrier Systems 21 (6) (2004), 477-520
- US 4,327,725, US 4,765,989, US 20030161882, EP 1 024 793.

Beschichtete osmotische Freisetzungssysteme sind ebenfalls bekannt. So beschreibt EP 0 339 811 ein osmotisches Freisetzungssystem bestehend aus einer Celluloseacetat-Hülle, die Nifedipin und Quellmittel im Kern enthält, und von einem Mantelüberzug aus HPMC (Hydroxypropylmethylcellulose) mit einer Schichtdicke von 0,0025 cm umgeben ist. US 4,948,592, WO 93/03711 und WO 93/00071 beschreiben osmotische Freisetzungssysteme enthaltend einen Wirkstoffanteil im Kern mit retardierendem Freisetzungsprofil und einen Anteil des gleichen Wirkstoffs im Mantelüberzug, der direkt freigesetzt werden kann. Dabei enthalten die Mantelüberzüge immer nur einen kleinen Teil der Gesamtwirkstoffmenge, die für die pharmazeutische Wirkung notwendig ist. Wirkstoffkombinationen mit unterschiedlichen Wirkstoffen oder Mantelüberzüge enthaltend Angiotensin-II Antagonisten und/oder ein Diuretikum werden nicht beschrieben.

Aufgabe der vorliegenden Erfindung ist die Bereitstellung einer stabilen pharmazeutischen Formulierung enthaltend Nifedipin oder Nisoldipin und mindestens einen Angiotensin-II Antagonisten und/oder mindestens ein Diuretikum, wobei der Angiotensin-II Antagonist und/oder das Diuretikum schnell (IR) und Nifedipin oder Nisoldipin verzögert (Retard) freigesetzt werden und somit dem Freisetzungsverhalten der bekannten Einzelformulierungen entspricht.

Überraschenderweise ist durch die vorliegende Erfindung die Bereitstellung einer stabilen pharmazeutischen Darreichungsform möglich, die einen Angiotensin-II Antagonisten und/oder ein Diuretikum in einer für dessen pharmazeutische Wirkung ausreichenden Menge enthält und diesen schnell freigeben kann, und die Nifedipin oder Nisoldipin enthält und kontrolliert (modifiziert) freigeben kann. Um die schnelle Freisetzung des Angiotensin-II Antagonisten und/oder des Diuretikums zu gewährleisten, ist es notwendig, diesen in die äußere Mantelschicht der Darreichungsform einzubringen. Aufgrund der notwendigen Menge an Wirkstoff erfordert dieses eine weitaus dickere Mantelschicht als die bislang bekannten Filmschichten osmotischer Freisetzungssysteme, die keinen Wirkstoff enthalten. Das Verhalten der äußeren Mantelschicht wird somit stark durch die Eigenschaften des verarbeiteten Wirkstoffs geprägt. Speziell bei osmotischen Wirkstofffreisetzungssystemen auf Celluloseacetat-Basis ist das Aufbringen dicker Schichten aufgrund der glatten und hydrophoben Oberfläche kritisch. Auch ist das generelle Problem der schlechten Kohärenz dicker Schichten durch die vorliegende Erfindung überraschenderweise gelöst und ein Abplatzen der Mantelschicht ist nicht zu beobachten. Die Anforderung, dass einheitliche Darreichungsformen hinsichtlich der Menge an Wirkstoff in der Mantelschicht (content uniformity) bereitgestellt werden, wird ebenfalls erfüllt. Dieses ist umso schwieriger, je dicker die Mantelschicht ist, da normalerweise bei Zunahme der Schichtdicke immer weiter zunehmende Schwankungen auftreten. Weiterhin erfordern dicke Mantelschichten lange Prozesszeiten unter feucht-warmen Bedingungen, die chemische Zersetzungsreaktionen des Wirkstoffs in der Mantelschicht beschleunigen können. Überraschenderweise wird durch die erfindungsgemäße Darreichungsform ein Freisetzungsverhalten der Wirkstoffe erzielt, das dem der bekannten Einzelformulierungen ungefähr entspricht, d.h. schnelle Freisetzung (IR) bei den Angiotensin-II Antagonisten und/oder Diuretika und kontrollierte (modifizierte, retardierte) bei Nifedipin oder Nisoldipin. Die erfindungsgemäßen Darreichungsformen können somit als bioäquivalent zu den bekannten Einzelformulierungen gleicher Dosis betrachtet werden. Weiterhin ist während des Aufsprühvorgangs gemäß erfinderischem Herstellungsverfahren die Stabilität des Angiotensin-II Antagonisten und/oder Diuretikums überraschenderweise gewährleistet.

Gegenstand der Erfindung ist eine pharmazeutische Darreichungsform enthaltend eine Wirkstoffkombination von Nifedipin oder Nisoldipin und mindestens einem Angiotensin-II Antagonisten und/oder mindestens ein Diuretikum und mindestens ein Film bildendes Polymer, dadurch gekennzeichnet, dass sich Nifedipin oder Nisoldipin im Kern und der Angiotensin-II Antagonist und/oder das Diuretikum in einem Mantelüberzug um den Kern befinden.

Weiterer Gegenstand der Erfindung ist eine pharmazeutische Darreichungsform, dadurch gekennzeichnet, dass das filmbildende Polymer partiell hydrolysierter Polyvinylalkohol ist.

Weiterer Gegenstand der Erfindung ist eine pharmazeutische Darreichungsform, dadurch gekennzeichnet, dass als filmbildendes Polymer ein kommerziell erhältliches Präparat verwendet wird, sogenannter "Fertiglack", der bereits weitere pharmazeutische Hilfsstoffe enthält und einfach in Wasser aufgelöst wird.

Weiterer Gegenstand der Erfindung ist eine pharmazeutische Darreichungsform, dadurch gekennzeichnet, dass als filmbildendes Polymer Opadry II 85F19250 Clear (Colorcon PVA-basierter Fertiglack) verwendet wird mit der Zusammensetzung: partiell hydrolysierter Polyvinylalkohol, Talkum, Polyethylenglykol (PEG 3350), Polysorbat 80 (Tween 80).

Weiterer Gegenstand der Erfindung ist eine pharmazeutische Darreichungsform enthaltend eine Wirkstoffkombination von Nifedipin oder Nisoldipin und mindestens einem Angiotensin-II Antagonisten und mindestens ein Film bildendes Polymer, dadurch gekennzeichnet, dass sich Nifedipin oder Nisoldipin im Kern und der Angiotensin-II Antagonist in einem Mantelüberzug um den Kern befinden.

Weiterer Gegenstand der Erfindung ist eine pharmazeutische Darreichungsform enthaltend eine Wirkstoffkombination von Nifedipin oder Nisoldipin und mindestens ein Diuretikum und mindestens ein Film bildendes Polymer, dadurch gekennzeichnet, dass sich Nifedipin oder Nisoldipin im Kern und das Diuretikum in einem Mantelüberzug um den Kern befinden.

Weiterer Gegenstand der Erfindung ist eine pharmazeutische Darreichungsform enthaltend eine Wirkstoffkombination von Nifedipin oder Nisoldipin einem Angiotensin-II Antagonisten und mindestens ein Diuretikum und mindestens ein Film bildendes Polymer, dadurch gekennzeichnet, dass sich Nifedipin oder Nisoldipin im Kern und der Angiotensin-II Antagonist und das Diuretikum in einem Mantelüberzug um den Kern befinden.

Die erfindungsgemäße Darreichungsform zeichnet sich dadurch aus, dass Nifedipin oder Nisoldipin retardiert und der Angiotensin-II Antagonist bzw. das Diuretikum schnell freigesetzt werden.

Weiterer Gegenstand der Erfindung ist eine pharmazeutische Darreichungsform, dadurch gekennzeichnet, dass die pharmazeutische Darreichungsform fest, oral applizierbar und auf Basis eines osmotischen Wirkstofffreisetzungssystems aufgebaut ist.

Weiterer Gegenstand der Erfindung ist eine pharmazeutische Darreichungsform, dadurch gekennzeichnet, dass die Wirkstoffe in kristalliner bzw. überwiegend kristalliner Form vorliegen.

Weiterer Gegenstand der Erfindung ist eine pharmazeutische Darreichungsform, dadurch gekennzeichnet, dass die Wirkstoffe in mikronisierter Form vorliegen.

Weiterer Gegenstand der Erfindung ist eine pharmazeutische Darreichungsform, dadurch gekennzeichnet, dass die Wirkstoffe ganz oder teilweise in amorpher Form vorliegen.

Weiterer Gegenstand der Erfindung ist eine pharmazeutische Darreichungsform, dadurch gekennzeichnet, dass Nifedipin oder Nisoldipin in einer Mindestdosis von 5 mg und einer Maximaldosis von 90 mg eingesetzt wird.

Weiterer Gegenstand der Erfindung ist eine pharmazeutische Darreichungsform, dadurch gekennzeichnet, dass Nifedipin in einer Mindestdosis von 10 mg und einer Maximaldosis von 60 mg und Nisoldipin in einer Mindestdosis von 5 mg und einer Maximaldosis von 30 mg eingesetzt wird.

Weiterer Gegenstand der Erfindung ist eine pharmazeutische Darreichungsform, dadurch gekennzeichnet, dass Nifedipin in einer Dosis von 20 mg, 30 mg oder 60 mg und Nisoldipin in einer Mindestdosis von 5 mg und einer Maximaldosis von 30 mg eingesetzt wird.

Weiterer Gegenstand der Erfindung ist eine pharmazeutische Darreichungsform, dadurch gekennzeichnet, dass Nifedipin oder Nisoldipin in einer Mindestdosis von 10 mg und einer Maximaldosis von 40 mg eingesetzt wird.

Weiterer Gegenstand der Erfindung ist eine pharmazeutische Darreichungsform, dadurch gekennzeichnet, dass der Angiotensin-II Antagonist Azilsartan, Candesartan, Losartan, Telmisartan, Irbesartan, Embursartan, Eprosartan, Valsartan oder Olmesartan, wobei Candesartan in Form von Candesartan cilexetil und Olmesartan in Form von Olmesartan medoxomil verwendet wird oder ein pharmazeutisch geeignetes Salz davon.

Weiterer Gegenstand der Erfindung ist eine pharmazeutische Darreichungsform, dadurch gekennzeichnet, dass der Angiotensin-II Antagonist Candesartan, Olmesartan, oder Telmisartan wobei Candesartan in Form von Candesartan cilexetil und Olmesartan in Form von Olmesartan medoxomil verwendet wirdoder ein pharmazeutisch geeignetes Salz davon.

Weiterer Gegenstand der Erfindung ist eine pharmazeutische Darreichungsform, dadurch gekennzeichnet, dass der Angiotensin-II Antagonist Candesartan oder Telmisartan oder ein Prodrug davon oder ein pharmazeutisch geeignetes Salz davon ist.

Weiterer Gegenstand der Erfindung ist eine pharmazeutische Darreichungsform, dadurch gekennzeichnet, dass der Angiotensin-II Antagonist Candesartan cilexetil ist.

Weiterer Gegenstand der Erfindung ist eine pharmazeutische Darreichungsform, dadurch gekennzeichnet, dass als Angiotensin-II Antagonist Candesartan oder Candesartan cilexetil in einer Dosis von 4-16 mg eingesetzt wird.

Weiterer Gegenstand der Erfindung ist eine pharmazeutische Darreichungsform, dadurch gekennzeichnet, dass als Angiotensin-II Antagonist Candesartan oder Candesartan cilexetil in einer Dosis von 2-32 mg eingesetzt wird.

Weiterer Gegenstand der Erfindung ist eine pharmazeutische Darreichungsform, dadurch gekennzeichnet, dass als Angiotensin-II Antagonist Candesartan oder Candesartan cilexetil in einer Dosis von 4 mg, 8 mg, 16 mg oder 32 mg eingesetzt wird.

Weiterer Gegenstand der Erfindung ist eine pharmazeutische Darreichungsform, dadurch gekennzeichnet, dass als Angiotensin-II Antagonist Candesartan oder Candesartan cilexetil in einer Dosis von 4-16 mg eingesetzt wird.

Weiterer Gegenstand der Erfindung ist eine pharmazeutische Darreichungsform, dadurch gekennzeichnet, dass der Angiotensin-II Antagonist Olmesartan oder Candesartan cilexetil in einer Dosis von 5-40 mg eingesetzt wird.

Weiterer Gegenstand der Erfindung ist eine pharmazeutische Darreichungsform, dadurch gekennzeichnet, dass Olmesartan oder Olmesartan medoxomil in einer Dosis von 10 bis 40 mg eingesetzt wird.

Weiterer Gegenstand der Erfindung ist eine pharmazeutische Darreichungsform, dadurch gekennzeichnet, dass der Angiotensin-II Antagonist Telmisartan ist und in einer Dosis von 10 bis 80 mg eingesetzt wird.

Weiterer Gegenstand der Erfindung ist eine pharmazeutische Darreichungsform, dadurch gekennzeichnet, dass Telmisartan in einer Dosis von 10 bis 40 mg eingesetzt wird.

Weiterer Gegenstand der Erfindung ist eine pharmazeutische Darreichungsform, dadurch gekennzeichnet, dass Telmisartan in einer Dosis von 20 mg, 40 mg oder 80 mg eingesetzt wird.

Weiterer Gegenstand der Erfindung ist eine pharmazeutische Darreichungsform, dadurch gekennzeichnet, dass Losartan in einer Dosis von 25 bis 100 mg eingesetzt wird.

Weiterer Gegenstand der Erfindung ist eine pharmazeutische Darreichungsform, dadurch gekennzeichnet, dass Losartan in einer Dosis von 40 bis 60 mg eingesetzt wird.

Weiterer Gegenstand der Erfindung ist eine pharmazeutische Darreichungsform, dadurch gekennzeichnet, dass Irbesartan in einer Dosis von 50 bis 500 mg eingesetzt wird.

Weiterer Gegenstand der Erfindung ist eine pharmazeutische Darreichungsform, dadurch gekennzeichnet, dass Irbesartan in einer Dosis von 75 bis 300 mg eingesetzt wird.

Weiterer Gegenstand der Erfindung ist eine pharmazeutische Darreichungsform, dadurch gekennzeichnet, dass das Diuretikum Hydrochlorothiazid, Chlorthalidon, Mefrusid, Piretanid oder Indapamid ist.

Weiterer Gegenstand der Erfindung ist eine pharmazeutische Darreichungsform, dadurch gekennzeichnet, dass das Diuretikum Hydrochlorothiazid oder Chlorthalidon ist.

Weiterer Gegenstand der Erfindung ist eine pharmazeutische Darreichungsform bestehend aus einem osmotischen Einkammersystem.

Weiterer Gegenstand der Erfindung ist eine pharmazeutische Darreichungsform bestehend aus einem osmotischen Einkammersystem umfassend
einen Kern, enthaltend
   - 5 bis 50 % des Wirkstoffes Nifedipin oder Nisoldipin,
   - 10 bis 50 % Xanthan,
   - 5 bis 40 % eines Vinylpyrrolidon-Vinylacetat-Copolymers,
sowie eine Hülle, bestehend aus einem wasserdurchlässigen, für die Komponenten des Kerns undurchlässigen Material mit mindestens einer Öffnung.

Weiterer Gegenstand der Erfindung ist eine pharmazeutische Darreichungsform, die im Kern zusätzlich Natriumchlorid als osmotisch aktiven Zusatz enthält.

Weiterer Gegenstand der Erfindung ist eine pharmazeutische Darreichungsform, die im Kern zusätzlich Natriumchlorid als osmotisch aktiven Zusatz in einer Menge bis 30% bezogen auf das Gesamtgewicht der inneren Kernbestandteile enthält.

Weiterer Gegenstand der Erfindung ist eine pharmazeutische Darreichungsform, dadurch gekennzeichnet, dass die Hülle aus Celluloseacetat oder einem Gemisch von Celluloseacetat und Polyethylenglykol besteht.

Weiterer Gegenstand der Erfindung ist ein Verfahren zur Herstellung eines osmotischen Einkammersystems, dadurch gekennzeichnet, dass die Komponenten des Kerns miteinander vermischt, granuliert und tablettiert werden, der so entstandene Kern mit einer Hülle beschichtet wird und die Hülle abschließend mit einer oder mehreren Öffnungen versehen wird.

Weiterer Gegenstand der Erfindung ist ein Verfahren zur Herstellung eines osmotischen Einkammersystems, dadurch gekennzeichnet, dass feucht granuliert wird.

Weiterer Gegenstand der Erfindung ist eine pharmazeutische Darreichungsform, bestehend aus einem osmotischen Zweikammersystem.

Weiterer Gegenstand der Erfindung ist eine pharmazeutische Darreichungsform, bestehend aus einem osmotischen Zweikammersystem umfassend
einen Kern mit einer Wirkstoffschicht, enthaltend
   - 5 bis 50 % des Wirkstoffes Nifedipin oder Nisoldipin,
   - 40 bis 95 % eines oder mehrerer osmotisch aktiver Polymere, und eine Osmoseschicht, enthaltend
   - 40 bis 90 % von einem oder mehreren osmotisch aktiven Polymeren
   - 5 bis 40 % eines osmotisch aktiven Zusatzes,
sowie eine Hülle, bestehend aus einem wasserdurchlässigen, für die Komponenten des Kerns undurchlässigen Material mit mindestens einer Öffnung.

Weiterer Gegenstand der Erfindung ist eine pharmazeutische Darreichungsform, die im Kern zusätzlich Natriumchlorid als osmotisch aktiven Zusatz enthält.

Weiterer Gegenstand der Erfindung ist eine pharmazeutische Darreichungsform, die im Kern in der Wirkstoffschicht Polyethylenoxid mit einer Viskosität von 40 bis 100 mPa·s (5 %ige wässrige Lösung, 25°C) als osmotisch aktives Polymer enthält und im Kern in der Osmoseschicht Polyethylenoxid mit einer Viskosität von 5000 bis 8000 mPa·s (1 %ige wässrige Lösung, 25°C) als osmotisch aktives Polymer enthält.

Weiterer Gegenstand der Erfindung ist eine pharmazeutische Darreichungsform, dadurch gekennzeichnet, dass die Hülle aus Celluloseacetat oder einem Gemisch von Celluloseacetat und Polyethylenglykol besteht.

Weiterer Gegenstand der Erfindung ist ein Verfahren zur Herstellung eines osmotischen Zweikammersystems, dadurch gekennzeichnet, dass
- die Komponenten der Wirkstoffschicht gemischt und granuliert werden und
- die Komponenten der Osmoseschicht gemischt und granuliert werden,
- anschließend auf einer Zweischichttablettenpresse beide Granulate zu einer Zweischichttablette verpresst werden,
- der so entstandene innere Kern dann mit der Hülle beschichtet wird und
- die Hülle auf der Wirkstoffseite mit einer oder mehreren Öffnungen versehen wird.

Weiterer Gegenstand der Erfindung ist ein Verfahren zur Herstellung eines osmotischen Zweikammersystems, dadurch gekennzeichnet, dass die Komponenten der Wirkstoffschicht trocken granuliert werden.

Weiterer Gegenstand der Erfindung ist ein Arzneimittel, enthaltend eine pharmazeutische Darreichungsform.

Weiterer Gegenstand der Erfindung ist die Verwendung einer pharmazeutischen Darreichungsform, zur Prophylaxe, Sekundärprophylaxe und/oder Behandlung von Erkrankungen.

Weiterer Gegenstand der Erfindung ist die Verwendung einer pharmazeutischen Darreichungsform, zur Herstellung eines Arzneimittels zur Prophylaxe, Sekundärprophylaxe und/oder Behandlung von Erkrankungen.

Weiterer Gegenstand der Erfindung ist die Verwendung einer pharmazeutischen Darreichungsform zur Prophylaxe, Sekundärprophylaxe und/oder Behandlung von Herz-/Kreislauf-Erkrankungen.

Weiterer Gegenstand der Erfindung ist die Verwendung einer pharmazeutischen Darreichungsform zur Prophylaxe, Sekundärprophylaxe und/oder Behandlung von Bluthochdruck.

Weiterer Gegenstand der Erfindung ist die Verwendung einer Nifedipin oder Nisoldipin/ und eines Angiotensin-II Antagonisten und/oder eines Diuretikums zur Herstellung einer pharmazeutischen Darreichungsform.

Weiterer Gegenstand der Erfindung ist die pharmazeutische Darreichungsform, in denen neben Nifedipin oder Nisoldipin und dem Angiotensin-II Antagonist ein weiterer antihypertensiver Wirkstoff eingearbeitet wird.

Weiterer Gegenstand der Erfindung ist die pharmazeutische Darreichungsform, in denen neben Nifedipin oder Nisoldipin und dem Angiotensin-II Antagonist ein Diuretikum eingearbeitet wird.

Weiterer Gegenstand der Erfindung ist die pharmazeutische Darreichungsform, wobei Hydrochlorothiazid eingesetzt wird.

Der Kern der erfindungsgemäßen pharmazeutischen Darreichungsform ist ein osmotisches Wirkstofffreisetzungssystem, das von dem erfindungsgemäßen Mantelüberzug enthaltend mindestens einen Angiotensin-II Antagonisten und/oder mindestens ein Diuretikum überzogen ist. Ein bevorzugter Gegenstand der Erfindung ist somit eine pharmazeutische Darreichungsform enthaltend eine Wirkstoffkombination von Nifedipin oder Nisoldipin und mindestens einem Angiotensin-II Antagonisten und/oder mindestens ein Diuretikum, dadurch gekennzeichnet, dass Nifedipin oder Nisoldipin sich im Kern eines osmotischen Wirkstofffreisetzungssystems und der Angiotensin-II Antagonist und/oder das Diuretikum sich in einem Mantelüberzug über dem osmotischen Wirkstofffreisetzungssystems befindet.

Ein weiterer Gegenstand der Erfindung ist eine pharmazeutische Darreichungsform enthaltend eine Wirkstoffkombination von Nifedipin oder Nisoldipin und mindestens einem Angiotensin-II Antagonisten und/oder mindestens ein Diuretikum, dadurch gekennzeichnet, dass Nifedipin oder Nisoldipin enthaltend im Kern kontrolliert (modifiziert) und der Angiotensin-II Antagonist und/oder das Diuretikum enthaltend in einem Mantelüberzug schnell im Körper freigegeben werden.

Als bevorzugte Angiotensin-II Antagonisten werden Azilsartan, Candesartan, Losartan, Telmisartan, Irbesartan, Embursartan, Eprosartan, Valsartan oder Olmesartan verwendet, besonders bevorzugt Candesartan, Olmesartan, oder Telmisartan, ganz besonders bevorzugt Candesartan oder Telmisartan oder Candesartan Cilexetil. Der Begriff "Prodrugs" umfasst Verbindungen, welche selbst biologisch aktiv oder inaktiv sein können, jedoch während ihrer Verweilzeit im Körper zu den erfindungsgemäß verwendeten Verbindungen umgesetzt werden (beispielsweise metabolisch oder hydrolytisch). Ein Prodrug von Candesartan ist beispielsweise Candesartan cilexetil. Dieses und weitere Beispiele für geeignete Prodrugs sind in J. Med. Chem. 1993 Aug 6;36(16):2343-9 offenbart. Ein Prodrug von Olmesartan ist beispielsweise Olmesartan medoxomil. Die genannten Sartane können auch in Form pharmazeutisch geeigneter Salze vorliegen, beispielsweise Losartan Kalium und Eprosartan Mesylat. In einer besonders bevorzugten Ausführungsform wird der Angiotensin-II Antagonist Candesartan cilexetil verwendet.

Als bevorzugte Diuretika werden Carboanhydrasehemmstoffe wie beispielsweise Acetazolamid, Dichlorphenamid und Methazolamid oder Schleifendiuretika wie beispielsweise Furosemid, Torasemid, Bumetanid, Etacrynsäure und Piretanid, oder kaliumsparende Diuretika wie beispielsweise Amilorid und Triamteren, oder Aldosteronantagonisten wie beispielsweise Spironolacton, Kaliumcanreonat und Eplerenon, oder Thiaziddiuretika und andere Sulfonamid-Diuretika wie beispielsweise Hydrochlorothiazid, Chlorthalidon, Xipamid, Metolazon, Mefrusid und Indapamid genannt. Besonders bevorzugt seien Hydrochlorothiazid, Chlorthalidon, Mefrusid, Piretanid und Indapamid genannt. Ganz besonders bevorzugt werden Hydrochlorothiazid oder Chlorthalidon verwendet.

Die erfindungsgemäße Darreichungsform enthält vorzugsweise Nifedipin oder Nisoldipin in Dosierungen von 5 bis 90 mg, im Fall von Nifedipin vorzugsweise in Dosierungen von 10 bis 60 mg, besonders bevorzugt in Dosierungen von 20, 30 oder 60 mg, im Fall von Nisoldipin vorzugsweise in Dosierungen von 5 bis 30 mg, und mindestens einen Angiotensin-II Antagonisten in Dosierungen von 2 bis 500 mg, vorzugsweise Candesartan, besonders bevorzugt Candesartan cilexetil in Dosierungen von 2 bis 40 mg, bevorzugt von 4 bis 32 mg, besonders bevorzugt in Dosierungen von 4, 8, 16 oder 32 mg, ebenfalls vorzugsweise Olmesartan, besonders bevorzugt Olmesartan medoxomil in einer Dosierung von 5 bis 40 mg, bevorzugt von 10 bis 40 mg, ebenfalls vorzugsweise Telmisartan in einer Dosierung von 10 bis 80 mg, besonders bevorzugt in einer Dosierung von 20, 40 oder 80 mg, ebenfalls vorzugsweise Losartan in einer Dosierung von 25 bis 100 mg, vorzugsweise von 40 bis 60 mg, ebenfalls vorzugsweise Azilsartan in einer Dosierung von 20 bis 80 mg, ebenfalls vorzugsweise Valsartan in einer Dosierung von 40 bis 320 mg, bevorzugt 80 bis 160 mg, ebenfalls vorzugsweise Irbesartan in einer Dosierung von 50 bis 500 mg, vorzugsweise von 75 bis 300 mg, ebenfalls vorzugsweise Eprosartan in einer Dosierung von 300 bis 600 mg, und/oder mindestens ein Diuretikum in Dosierungen von 0,5 bis 500 mg, vorzugsweise Hydrochlorothiazid (HCT, HCTZ) in Dosierungen von 12,5 bis 25 mg, ebenfalls vorzugsweise Chlorthalidon in Dosierungen von 12,5 mg bis 50 mg, ebenfalls vorzugsweise Metolazon in Dosierungen von 5 bis 10 mg, ebenfalls vorzugsweise Spironolacton in Dosierungen von 25 bis 100 mg. ebenfalls vorzugsweise Furosemid in Dosierungen von 20 bis 80 mg, ebenfalls vorzugsweise Mefrusid in einer Dosierung von 12,5 mg, ebenfalls vorzugsweise Piretanid in Dosierungen von 3 bis 6 mg, ebenfalls vorzugsweise Indapamid in Dosierungen von 0,5 bis 5 mg.

Besonders bevorzugte Dosiskombinationen sind alle 87 möglichen Kombinationen aus Nifedipin in den Dosen 20 mg, 30 mg und 60 mg mit Candesartan cilexetil in den Dosen 4 mg, 8 mg, 16 mg und 32 mg und/oder einem Diuretikum, ausgewählt aus Hydrochlorothiazid in den Dosen 12,5 mg und 25 mg und Chlorthalidon in den Dosen 12,5 mg, 25 mg und 50 mg.

Diese möglichen Kombinationen sind in den nachfolgenden Tabellen veranschaulicht (alle Angaben in mg):

**Tabelle a) Kombinationen aus zwei Wirkstoffen**

| Nr. | Nifedipin | Candesartan cilexetil | Hydrochlorothiazid | Chorthalidon |
|---|---|---|---|---|
| 1 | 20 | 4 | | |
| 2 | 20 | 8 | | |
| 3 | 20 | 16 | | |
| 4 | 20 | 32 | | |
| 5 | 20 | | 12,5 | |
| 6 | 20 | | 25 | |
| 7 | 20 | | | 12,5 |
| 8 | 20 | | | 25 |
| 9 | 20 | | | 50 |
| 10 | 30 | 4 | | |
| 11 | 30 | 8 | | |
| 12 | 30 | 16 | | |
| 13 | 30 | 32 | | |
| 14 | 30 | | 12,5 | |
| 15 | 30 | | 25 | |
| 16 | 30 | | | 12,5 |
| 17 | 30 | | | 25 |
| 18 | 30 | | | 50 |
| 19 | 60 | 4 | | |
| 20 | 60 | 8 | | |
| 21 | 60 | 16 | | |
| 22 | 60 | 32 | | |
| 23 | 60 | | 12,5 | |
| 24 | 60 | | 25 | |
| 25 | 60 | | | 12,5 |
| 26 | 60 | | | 25 |
| 27 | 60 | | | 50 |

**Tabelle b) Kombinationen aus drei Wirkstoffen**

| Nr. | Nifedipin | Candesartan cilexetil | Hydrochlorothiazid | Chorthalidon |
|---|---|---|---|---|
| 28 | 20 | 4 | 12,5 | |
| 29 | 20 | 4 | 25 | |
| 30 | 20 | 4 | | 12,5 |
| 31 | 20 | 4 | | 25 |
| 32 | 20 | 4 | | 50 |
| 33 | 20 | 8 | 12,5 | |
| 34 | 20 | 8 | 25 | |
| 35 | 20 | 8 | | 12,5 |
| 36 | 20 | 8 | | 25 |
| 37 | 20 | 8 | | 50 |
| 38 | 20 | 16 | 12,5 | |
| 39 | 20 | 16 | 25 | |
| 40 | 20 | 16 | | 12,5 |
| 41 | 20 | 16 | | 25 |
| 42 | 20 | 16 | | 50 |
| 43 | 20 | 32 | 12,5 | |
| 44 | 20 | 32 | 25 | |
| 45 | 20 | 32 | | 12,5 |
| 46 | 20 | 32 | | 25 |
| 47 | 20 | 32 | | 50 |
| 48 | 30 | 4 | 12,5 | |
| 49 | 30 | 4 | 25 | |
| 50 | 30 | 4 | | 12,5 |
| 51 | 30 | 4 | | 25 |
| 52 | 30 | 4 | | 50 |
| 53 | 30 | 8 | 12,5 | |
| 54 | 30 | 8 | 25 | |
| 55 | 30 | 8 | | 12,5 |
| 56 | 30 | 8 | | 25 |
| 57 | 30 | 8 | | 50 |
| 58 | 30 | 16 | 12,5 | |
| 59 | 30 | 16 | 25 | |
| 60 | 30 | 16 | | 12,5 |
| 61 | 30 | 16 | | 25 |
| 62 | 30 | 16 | | 50 |
| 63 | 30 | 32 | 12,5 | |
| 64 | 30 | 32 | 25 | |
| 65 | 30 | 32 | | 12,5 |
| 66 | 30 | 32 | | 25 |
| 67 | 30 | 32 | | 50 |
| 68 | 60 | 4 | 12,5 | |
| 69 | 60 | 4 | 25 | |
| 70 | 60 | 4 | | 12,5 |
| 71 | 60 | 4 | | 25 |
| 72 | 60 | 4 | | 50 |
| 73 | 60 | 8 | 12,5 | |
| 74 | 60 | 8 | 25 | |
| 75 | 60 | 8 | | 12,5 |
| Nr. | Nifedipin | Candesartan cilexetil | Hydrochlorothiazid | Chorthalidon |
| 76 | 60 | 8 | | 25 |
| 77 | 60 | 8 | | 50 |
| 78 | 60 | 16 | 12,5 | |
| 79 | 60 | 16 | 25 | |
| 80 | 60 | 16 | | 12,5 |
| 81 | 60 | 16 | | 25 |
| 82 | 60 | 16 | | 50 |
| 83 | 60 | 32 | 12,5 | |
| 84 | 60 | 32 | 25 | |
| 85 | 60 | 32 | | 12,5 |
| 86 | 60 | 32 | | 25 |
| 87 | 60 | 32 | | 50 |

Weiterhin besonders bevorzugte Dosiskombinationen sind alle möglichen Zweier- und DreierKombinationen aus Nifedipin in den Dosen 20 mg, 30 mg und 60 mg mit Telmisartan in den Dosen 20 mg, 40 mg, und 80 mg und/oder einem Diuretikum, ausgewählt aus Hydrochlorothiazid in den Dosen 12,5 mg und 25 mg und Chlorthalidon in den Dosen 12,5 mg, 25 mg und 50 mg.

Bevorzugt befindet sich die komplette Wirkstoffmenge an Nifedipin oder Nisoldipin im Kern, bevorzugt im Kern des osmotischen Freisetzungssystems, und die komplette Wirkstoffmenge an Angiotensin-II Antagonisten und/oder Diuretikum im Mantelüberzug. In Ausführungsformen, die einen Angiotensin-II-Antagonisten und ein Diuretikum im Mantelüberzug enthalten, können sich der Angiotensin-II-Antagonisten und das Diuretikum in derselben Schicht des Mantelüberzugs oder in voneinander getrennten, nacheinander aufgebrachten Schichten des Mantelüberzugs befinden.

Gegebenenfalls kann es erforderlich sein, von den genannten Mengen abzuweichen, und zwar in Abhängigkeit vom Körpergewicht bzw. der Art des Applikationsweges, vom individuellen Verhalten gegenüber den Medikamenten, der Art von deren Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muss.

Ein weiterer Gegenstand der Erfindung sind feste, oral applizierbare, eine Wirkstoffkombination von Nifedipin oder Nisoldipin mit einem Angiotensin-II Antagonisten und/oder einem Diuretikum enthaltende pharmazeutische Darreichungsformen zur einmal täglichen Applikation basierend auf osmotischen Abgabesystemen, dadurch gekennzeichnet, dass mindestens 85 % des Nifedipins oder Nisoldipins (bezogen auf die deklarierte Gesamtmenge des jeweiligen Wirkstoffes) über einen Zeitraum von 24 Stunden, bevorzugt 5 bis 17 % des Nifedipins oder Nisoldipins innerhalb von 4 Stunden und 43 bis 80 % des Nifedipins oder Nisoldipins innerhalb von 12 Stunden freigesetzt werden gemäß USP-Freisetzungsmethode mit Apparatur 2 (Paddle) bei 100 Umdrehungen pro Minute in 900 ml Phosphatpuffer pH 6,8 mit Zusatz von 1% Natriumlaurylsulfat bei 37°C und mindestens 70 % des Angiotensin-II Antagonisten und/oder des Diuretikums (bezogen auf die deklarierte Gesamtmenge des jeweiligen Wirkstoffes) innerhalb von 30 Minuten freigesetzt werden gemäß USP-Freisetzungsmethode mit Apparatur 2 (Paddle) bei 75 Umdrehungen pro Minute in 1000 ml eines geeigneten Mediums, beispielsweise Phosphatpuffer bei 37°C.

Ein weiterer Gegenstand der Erfindung sind feste, oral applizierbare, eine Wirkstoffkombination von Nifedipin oder Nisoldipin mit einem Angiotensin-II Antagonisten und/oder einem Diuretikum enthaltende pharmazeutische Darreichungsformen zur einmal täglichen Applikation basierend auf osmotischen Abgabesystemen, dadurch gekennzeichnet, dass mindestens 85 % des Nifedipins oder Nisoldipins (bezogen auf die deklarierte Gesamtmenge des jeweiligen Wirkstoffes) über einen Zeitraum von 24 Stunden, bevorzugt 5 bis 17 % des Nifedipins oder Nisoldipins innerhalb von 4 Stunden und 43 bis 80 %, besonders bevorzugt 45 bis 75 % des Nifedipins oder Nisoldipins innerhalb von 12 Stunden, sowie mindestens 60%, bevorzugt mindestens 70 %, besonders bevorzugt mindestens 80 % des Angiotensin-II Antagonisten und/oder des Diuretikums (bezogen auf die deklarierte Gesamtmenge des jeweiligen Wirkstoffes) innerhalb von 30 Minuten freigesetzt werden gemäß USP-Freisetzungsmethode mit Apparatur 2 (Paddle) bei 75 Umdrehungen pro Minute in 900 ml geeignetem Mediums, beispielsweise 0,1 N Salzsäure (pH 1,0) mit Zusatz von 1,0 % Natriumlaurylsulfat oder Phosphatpuffer pH 6,8 mit Zusatz von 1,0 % Natriumlaurylsulfat bei 37°C).

Dem Fachmann ist geläufig, dass die Bedingungen des Freisetzungstests an die Löslichkeiten des Wirkstoffs angepasst werden müssen. Für Formulierungen, die Nifedipin und Candesartan cilexetil enthalten, wird die Prüfung auf in-vitro Dissolution beispielsweise bevorzugt für beide Wirkstoffe simultan gemäß USP-Freisetzungsmethode mit Apparatur 2 (Paddle), bei 75 Umdrehungen in 900 mL eines geeigneten Mediums bei 37°C durchgeführt, wobei das geeignete Medium ausgewählt wird aus 0,1 N Salzsäure (pH 1,0), 0,01 N Salzsäure (pH 2,0), Acetatpuffer pH 4,5, Phosphatpuffer pH 6,5, Phosphatpuffer pH 6,8 und Phosphatpuffer pH 8,0, jeweils mit einem Zusatz von 0,6 - 1,0 % Natriumlaurylsulfat oder 0,6-1,0 % Polysorbat 20 (Tween 20). Besonders bevorzugt ist 0,1 N Salzsäure (pH 1,0) mit einem Zusatz von 1,0 % Natriumlaurylsulfat.

Ein weiterer Gegenstand der Erfindung sind feste, oral applizierbare, eine Wirkstoffkombination von Nifedipin mit Candesartan cilexetil und ggf. ein Diuretikum enthaltende pharmazeutische Darreichungsformen zur einmal täglichen Applikation basierend auf osmotischen Abgabesystemen, dadurch gekennzeichnet, dass mindestens 85 % des Nifedipins (bezogen auf die deklarierte Gesamtmenge des Nifedipins) über einen Zeitraum von 24 Stunden, 5 bis 17 % des Nifedipins innerhalb von 4 Stunden und 45 bis 75 % des Nifedipins innerhalb von 12 Stunden, sowie mindestens 70 % des Candesartan cilexetils (bezogen auf die deklarierte Gesamtmenge des Candesartan cilexetils) innerhalb von 30 Minuten freigesetzt werden gemäß USP-Freisetzungsmethode mit Apparatur 2 (Paddle) bei 75 Umdrehungen pro Minute in 900 ml 0,1 N Salzsäure (pH 1,0) mit einem Zusatz von 1,0 % Natriumlaurylsulfat bei 37°C.

Ein weiterer Gegenstand der Erfindung sind feste, oral applizierbare, eine Wirkstoffkombination von Nifedipin mit Telmisartan und ggf. ein Diuretikum enthaltende pharmazeutische Darreichungsformen zur einmal täglichen Applikation basierend auf osmotischen Abgabesystemen, dadurch gekennzeichnet, dass mindestens 85 % des Nifedipins (bezogen auf die deklarierte Gesamtmenge des Nifedipins) über einen Zeitraum von 24 Stunden, 5 bis 17 % des Nifedipins innerhalb von 4 Stunden und 43 bis 80 % des Nifedipins innerhalb von 12 Stunden, sowie mindestens 60 % des Telmisartans (bezogen auf die deklarierte Gesamtmenge des Telmisartans) innerhalb von 30 Minuten freigesetzt werden gemäß USP-Freisetzungsmethode mit Apparatur 2 (Paddle) bei 75 Umdrehungen pro Minute in 900 ml Acetatpuffer pH 4,5 mit einem Zusatz von 0,6 % Natriumlaurylsulfat bei 37°C.

Ein weiterer Gegenstand der Erfindung sind feste, oral applizierbare, eine Wirkstoffkombination von Nifedipin oder Nisoldipin mit einem Angiotensin-II Antagonisten und/oder einem Diuretikum enthaltende pharmazeutische Darreichungsformen zur einmal täglichen Applikation basierend auf osmotischen Abgabesystemen, dadurch gekennzeichnet, dass sich das Freisetzungsprofil von Nifedipin um weniger als 30 Minuten, bevorzugt um weniger als 15 Minuten, besonders bevorzugt um weniger als 5 Minuten gegenüber dem Freisetzungsprofil von Nifedipin in Adalat® GITS Formulierungen gleicher Dosis unterscheidet und das Freisetzungsprofil des Angiotensin-II Antagonisten und/oder des Diuretikums um weniger als 10 Minuten, bevorzugt weniger als 5 Minuten gegenüber dem Freisetzungsprofil des Angiotensin-II Antagonisten in der dem Wirkstoff entsprechenden vermarkteten Formulierung gleicher Dosis wie beispielsweise Candesartan cilexetil in Atacand® oder Blopress®, oder Telmisartan in Kinzalmono® oder Micardis®, oder Hydrochlorothiazid in Esidrix® unterscheidet.

Die Wirkstoffe können in den erfindungsgemäßen pharmazeutischen Darreichungsformen in kristalliner Form oder in nicht-kristalliner, amorpher Form vorliegen oder in Mischungen von kristallinen und amorphen Wirkstoffanteilen. Einige Wirkstoffe können in mehreren Kristallmodifikationen vorkommen. Prinzipiell können die erfindungsgemäßen pharmazeutischen Darreichungsformen die Wirkstoffe in allen möglichen Kristallformen enthalten. Beispielsweise ist bekannt, dass Candesartan cilexetil in der Modifikation I oder Typ C vorkommt (vgl. EP 0 459 136 B1). Es ist ferner bekannt, dass es von Candesartan cilexetil weitere Kristallmodifikationen gibt (vgl. z.B. WO 2008/035360 A). Bevorzugt wird Candesartan cilexetil der Kristallmodifikation I verwendet. Auch von Telmisartan ist eine polymorphe Kristallmodifikation B bekannt (vgl. WO 00/43370 A).

Zur Erzielung einer möglichst gleichförmigen Verteilung des Wirkstoffes wird dieser in der erfindungsgemässen Darreichungsformen in einer möglichst kleinen Partikelgröße eingesetzt. Dem Fachmann sind Methoden zur Verringerung der Partikelgröße geläufig. Darunter ist insbesondere die Feinstvermahlung mittels Luftstrahlmühle (Mikronisierung) bevorzugt. Mittels dieses Mahlverfahrens können typischerweise Partikelgrößenverteilungen erzielt werden, die sich durch einen X₅₀ Wert von 10 µm oder darunter und durch einen X₉₀ Wert von 30 µm oder darunter auszeichnen.

Enthalten die erfindungsgemäßen Darreichungsformen die Wirkstoffe in kristalliner Form, werden sie in einer bevorzugten Ausführungsform der vorliegenden Erfindung in mikronisierter Form eingesetzt, bevorzugt mit einem mittleren Partikeldurchmesser von kleiner 10 µm, bevorzugt kleiner 5 µm, besonders bevorzugt kleiner 3 µm. Hierbei weisen Nifedipin oder Nisoldipin vorzugsweise eine mittlere Partikelgröße X₅₀ von 2 bis 6 µm sowie einen X₉₀-Wert (90 %-Anteil) kleiner 12 µm auf. Candesartan cilexetil weist vorzugsweise eine mittlere Partikelgröße X₅₀ von 0,5 bis 8 µm, bevorzugt 1 bis 5 µm, sowie einen X₉₀-Wert (90 %-Anteil) kleiner 20 µm, bevorzugt kleiner 10 µm auf. Die Angaben X₅₀ und X₉₀ beziehen sich stets auf die Partikelgrößenverteilung bestimmt mittels Laserdiffraktometrie und angegeben als Volumenverteilung.

Für das osmotisches Wirkstofffreisetzungssystem sind sowohl osmotische Einkammersysteme (elementary osmotic pump) als auch Zweikammersysteme (push-pull-Systeme) geeignet.

Die Hülle des osmotisches Wirkstofffreisetzungssystem besteht sowohl beim Einkammer- als auch beim Zweikammersystem aus einem wasserdurchlässigen, für die Komponenten des inneren Kerns undurchlässigen Material. Solche Hüllmaterialien sind im Prinzip bekannt und beispielsweise beschrieben in der EP 024 793 B1, Seite 3-4, deren Offenbarung hiermit durch Bezugnahme eingeschlossen ist. Erfindungsgemäß bevorzugt werden als Hüllmaterial Celluloseacetat oder Gemische von Celluloseacetat und Polyethylenglykol eingesetzt.

Beim osmotischen Einkammersystem enthält der innere Kern vorzugsweise 5 bis 50 % Nifedipin oder Nisoldipin, 10 bis 50 % Xanthan und 5 bis 40 % eines Vinylpyrrolidon-Vinylacetat-Copolymers, wobei gegebenenfalls die Differenz zu 100 % durch einen oder mehrere zusätzliche Bestandteile gebildet wird, die ausgewählt sind aus der Gruppe von weiteren hydrophilen, quellbaren Polymeren, osmotisch aktiven Zusätzen und pharmazeutisch üblichen Hilfsstoffen. Die Summe der inneren Kernbestandteile beträgt 100 % und die %- Angaben beziehen sich jeweils auf das Gesamtgewicht des inneren Kerns.

Das osmotische Einkammersystem enthält als einen der wesentlichen Bestandteile des inneren Kerns das hydrophile wasserquellbare Polymer Xanthan. Dabei handelt es sich um ein anionisches Heteropolysaccharid, das im Handel beispielsweise unter der Bezeichnung Rhodigel® (hergestellt durch Rhodia) erhältlich ist. Es liegt in einer Menge von 10 bis 50 %, bevorzugt von 20 bis 40 %, bezogen auf das Gesamtgewicht der inneren Kernbestandteile vor.

Ein weiterer wesentlicher Bestandteil des inneren Kerns ist das Vinylpyrrolidon-Vinylacetat-Copolymer. Dieses Copolymer ist an sich bekannt und kann mit beliebigen Mischungsverhältnissen der Monomere hergestellt werden. Das bevorzugt verwendete kommerziell erhältliche Kollidon® VA64 (hergestellt durch BASF) ist z.B. ein 60:40- Copolymerisat. Es weist im allgemeinen einen Gewichtsmittelwert des Molekulargewichts Mw, bestimmt durch Lichtstreuungsmessungen, von etwa 45.000 bis etwa 70.000 auf. Die Menge des Vinylpyrrolidon-Vinylacetat-Copolymers im inneren Kern beträgt 5 bis 40%, bevorzugt 15 bis 25%, bezogen auf das Gesamtgewicht der inneren Kernbestandteile.

Gegebenenfalls im inneren Kern zusätzlich vorhandene hydrophile quellbare Polymere sind beispielsweise Hydroxypropylcellulose, Hydroxypropylmethylcellulose, Natriumcarboxymethylcellulose, Natriumcarboxymethylstärke, Polyacrylsäuren bzw. deren Salze.

Gegebenenfalls im inneren Kern zusätzlich vorhandene osmotisch aktive Zusätze sind beispielsweise alle wasserlöslichen Stoffe, deren Verwendung in der Pharmazie unbedenklich ist, wie z.B. die in Pharmakopöen oder in "Hager" und "Remington Pharmaceutical Science" erwähnten wasserlöslichen Hilfsstoffe. Insbesondere können wasserlösliche Salze von anorganischen oder organischen Säuren oder nichtionische organische Stoffe mit großer Wasserlöslichkeit wie z.B. Kohlehydrate, insbesondere Zucker, Zuckeralkohole oder Aminosäuren verwendet werden. Zum Beispiel können die osmotisch aktiven Zusätze ausgewählt werden aus anorganischen Salzen wie Chloriden, Sulfaten, Carbonaten und Bicarbonaten von Alkali- oder Erdalkalimetallen, wie Lithium, Natrium, Kalium, Magnesium, Calcium sowie Phosphate, Hydrogen- oder Dihydrogenphosphate, Acetate, Succinate, Benzoate, Citrate oder Ascorbate davon. Des weiteren können Pentosen, wie Arabinose, Ribose oder Xylose, Hexosen, wie Glucose, Fructose, Galactose oder Mannose, Disaccharide wie Sucrose, Maltose oder Lactose oder Trisaccharide wie Raffinose verwendet werden. Zu den wasserlöslichen Aminosäuren zählen Glycin, Leucin, Alanin oder Methionin. Erfindungsgemäß besonders bevorzugt wird Natriumchlorid verwendet. Die osmotisch aktiven Zusätze sind bevorzugt in einer Menge bis 30 % bezogen auf das Gesamtgewicht der inneren Kernbestandteile enthalten.

Gegebenenfalls im inneren Kern zusätzlich vorhandene pharmazeutisch übliche Hilfsstoffe sind beispielsweise Pufferstoffe wie Natriumbicarbonat, Bindemittel wie Hydroxypropylcellulose, Hydroxypropylmethylcellulose oder Polyvinylpyrrolidon, Schmiermittel wie Magnesiumstearat, Netzmittel wie Natriumlaurylsulfat oder Fließregulierungsmittel wie hochdisperses Siliziumdioxid und Stabilisatoren wie Antioxidantien.

Zur Herstellung des osmotischen Einkammersystems können beispielsweise die Komponenten des inneren Kerns umfassend Nifedipin oder Nisoldipin miteinander vermischt werden, gegebenenfalls feucht oder trocken, bevorzugt feucht granuliert werden, anschließend tablettiert werden, und der so entstandene innere Kern mit der Hülle beschichtet werden, die mit einer oder mehreren Öffnungen versehen wird.

Beim osmotischen Zweikammersystem besteht der innere Kern aus zwei Schichten, einer Wirkstoffschicht und einer Osmoseschicht. Ein derartiges osmotisches Zweikammersystem ist beispielsweise in DE 34 17 113 C2, deren Offenbarung hiermit durch Bezugnahme eingeschlossen ist, ausführlich beschrieben.

Die Wirkstoffschicht enthält vorzugsweise 5 bis 50 % Nifedipin oder Nisoldipin, bevorzugt 10 bis 45%, weiterhin bevorzugt 10 bis 40%, weiterhin bevorzugt 10 bis 30%, weiterhin besonders bevorzugt 15 bis 25%, insbesondere bevorzugt 18 bis 22%, ganz besonders bevorzugt 20% und 40 bis 95 %, bevorzugt 50 bis 85%, weiterhin bevorzugt 55 bis 85%, weiterhin bevorzugt 60 bis 85%, weiterhin ganz besonders bevorzugt 65 bis 85%, insbesondere bevorzugt 70 bis 80% von einem oder mehreren osmotisch aktiven Polymeren, vorzugsweise Polyethylenoxid mittlerer Viskosität (40 bis 100 mPa·s; 5 %ige wässrige Lösung, 25°C) und die Osmoseschicht enthält vorzugsweise 40 bis 90 %, bevorzugt 50 bis 80%, weiterhin bevorzugt 55 bis 75%, weiterhin bevorzugt 55 bis 70%, insbesondere 60 bis 67% von einem oder mehreren osmotisch aktiven Polymeren, vorzugsweise Polyethylenoxid hoher Viskosität (5000 bis 8000 mPa·s; 1 %ige wässrige Lösung, 25°C) und 5 bis 40 %, bevorzugt 10 bis 40%, weiterhin bevorzugt 15 bis 40%, weiterhin bevorzugt 20 bis 40%, insbesondere bevorzugt 20 bis 35% eines osmotisch aktiven Zusatzes, wobei die Differenz zu 100 % in den einzelnen Schichten unabhängig voneinander jeweils durch einen oder mehrere zusätzliche Bestandteile in Form von pharmazeutisch üblichen Hilfsstoffen gebildet wird. Die %-Angaben beziehen sich jeweils auf das Gesamtgewicht der jeweiligen inneren Kernschicht.

Im inneren Kern des osmotischen Zweikammersystems können weiterhin auch die gleichen osmotisch aktiven Zusätze wie im oben beschriebenen Fall des Einkammersystems verwendet werden. Bevorzugt ist hierbei Natriumchlorid.

Im inneren Kern des osmotischen Zweikammersystems können die gleichen pharmazeutisch üblichen Hilfsstoffe wie im oben beschriebenen Fall des Einkammersystems verwendet werden. Bevorzugt sind hierbei Bindemittel wie Hydroxypropylcellulose, Hydroxypropylmethylcellulose oder Polyvinylpyrrolidon, Schmiermittel wie Magnesiumstearat, Netzmittel wie Natriumlaurylsulfat oder Fließregulierungsmittel wie hochdisperses Siliziumdioxid, ein Farbpigment wie Eisenoxid in einer der beiden Schichten zur Differenzierung von Wirkstoff- und Osmoseschicht sowie Stabilisatoren/Antioxidantien in der Wirkstoffschicht.

Zur Herstellung des osmotischen Zweikammersystems können beispielsweise die Komponenten der Wirkstoffschicht umfassend Nifedipin oder Nisoldipin gemischt und feucht oder trocken, bevorzugt trocken granuliert werden, die Komponenten der Osmoseschicht gemischt und granuliert werden und anschließend beide Granulate auf einer Zweischichttablettenpresse zu einer Zweischichttablette verpresst werden. Der so entstandene innere Kern wird dann mit einer Hülle beschichtet. Die Hülle wird auf der Wirkstoffseite mit einer oder mehreren Öffnungen versehen. Alternativ dazu kann die Einführung der einen oder mehreren Öffnungen in diesem Prozessschritt unterbleiben. In diesem Fall werden erst nach erfolgter Beschichtung mit einem oder mehreren weiteren Mantelüberzügen beide Seiten der Tablette mit jeweils einer Öffnung versehen, die jeweils von außen bis zum inneren Kern reichen, d.h. die Mantelüberzüge und die Hülle durchqueren.

In einer bevorzugten Ausführungsform der vorliegenden Erfindung werden bei der Herstellung des osmotischen Zweikammersystems sowohl die Komponenten der Wirkstoffschicht als auch die Komponenten der Osmoseschicht jeweils granuliert, insbesondere mit Hilfe der Walzengranulation.

Erfindungsgemäß bevorzugt sind aufgrund der physikalisch-chemischen Eigenschaften der Wirkstoffkombination osmotische Zweikammersysteme (push-pull-Systeme), bei denen die Wirkstoff- und Osmoseschicht getrennt vorliegen, beispielhaft und vorzugsweise als 2-Schichttablette formuliert. Die Vorteile gegenüber osmotischen Einkammersystemen sind hierbei die über einen längeren Zeitraum gleichmäßigere Freisetzungsrate sowie die Möglichkeit, den systembedingt notwendigen Wirkstoffüberschuss zu verringern.

Der Mantelüberzug der erfindungsgemäßen Darreichungsform enthält mindestens einen Angiotensin-II Antagonisten und/oder mindestens ein Diuretikum und mindestens ein Film bildendes Polymer. Das Film bildende Polymer kann so gewählt werden, dass es geeignet für die schnelle Freisetzung von Wirkstoffen ist. In Ausführungsformen, die einen Angiotensin-II-Antagonisten und ein Diuretikum im Mantelüberzug enthalten, können sich der Angiotensin-II-Antagonisten und das Diuretikum in derselben Überzugsschicht oder in voneinander getrennten nacheinander aufgebrachten Überzugsschichten befinden.

Als Film bildendes Polymer wird partiell hydrolysierter Polyvinylalkohol verwendet.

Bevorzugt sind weiterhin insbesondere folgende kommerziell erhältlichen Präparate, sogenannte "Fertiglacke", die bereits weitere pharmazeutische Hilfsstoffe enthalten und einfach in Wasser aufgelöst werden.
- Kollicoat IR white (BASF PVA-co-PEG-basierter Fertiglack mit Weißpigment), Zusammensetzung: Kollicoat IR (PVA-co-PEG), Kollidon VA64 (Copovidone), Kaolin, Natriumlaurylsulfat, Titandioxid.
- Sepifilm IR Colorless (SEPPIC PVA-co-PEG-basierter Fertiglack ohne Pigmente), Zusammensetzung: Kollicoat IR (PVA-co-PEG), Polydextrose, Kaolin, Polyethylenglykol (PEG 400).
- Opadry II 85F19250 Clear (Colorcon PVA-basierter Fertiglack), Zusammensetzung: partiell hydrolysierter Polyvinylalkohol, Talkum, Polyethylenglykol (PEG 3350), Polysorbat 80 (Tween 80). Dieser Fertiglack ist besonders bevorzugt.
- Opadry II 85F28393 (Colorcon PVA-basierter Fertiglack), Zusammensetzung: partiell hydrolysierter Polyvinylalkohol, Talkum, Polyethylenglykol (PEG 3350), Titandioxid.

Der Mantelüberzug kann auch aus den Einzelkomponenten beispielsweise aus folgenden kommerziell erhältliche Präparaten hergestellt werden: BASF Kollicoat IR (PVA-co-PEG), BASF Kollidon VA64 (Copovidone), Merck Emprove (PVA).

Der Mantelüberzug kann weitere Hilfsstoffe enthalten wie beispielsweise Netzmittel (z. B. Natriumlaurylsulfat, quarternäre Ammoniumverbindungen, Lecithin (insbesondere Soja-Lecithin), Polysorbate (insbesondere Polysorbat 80, synonym Tween 80)), Pigmente (z. B. Titandioxid, Talkum), Farbpigmente (z. B. Eisenoxid rot, gelb oder schwarz oder Mischungen daraus), Trennmittel (z. B. Kaolin, Talkum, hochdisperses Siliciumdioxid, Magnesiumstearat, Glycerolmonostearat), und/oder Weichmacher (z. B. Polyethylenglykol (insbesondere Polyethylenglycol 400, Polyethylenglycol 3350), Polypropylenglycol, Propylenglycol, Glycerol, Triacetin, Triethylcitrat).

Im Mantelüberzug beträgt der Anteil des Angiotensin-II Antagonisten, gegebenenfalls zusammen mit dem Anteil des Diuretikums, 10 bis 50 %, bevorzugt 15 bis 40 %, bevorzugt 10 bis 40 %, besonders bevorzugt 20 bis 40%, weiterhin bevorzugt 20 bis 45 %, besonders bevorzugt 25 bis 45%, besonders bevorzugt 30 bis 45%, ganz besonders bevorzugt 20, 30, 33, 40% oder 45%, der Anteil des Film bildenden Polymers beträgt 20 bis 75 %, bevorzugt 25 bis 60 %, besonders bevorzugt etwa 30 bis 45 %, der Pigmentanteil beträgt 0 bis 20 %, der Anteil an Netzmittel beträgt 0 bis 3 %, bevorzugt 1 bis 2 %, bezogen auf das Trockengewicht des Mantelüberzugs. Bei der Verwendung von Fertiglacken beträgt der Anteil des Angiotensin-II Antagonisten, gegebenenfalls zusammen mit dem Anteil des Diuretikums, 10 bis 50 %, bevorzugt 15 bis 40 %, bevorzugt 10 bis 40 %, besonders bevorzugt 20 bis 40%, weiterhin bevorzugt 20 bis 45 %, besonders bevorzugt 25 bis 45%, besonders bevorzugt 30 bis 45%, ganz besonders bevorzugt 20, 30, 33 40% oder 45%, besonders bevorzugt 40 %, und der Anteil des Fertiglacks 50 bis 90 %, bevorzugt 50 bis 80 %, bevorzugt 50 bis 75 %, bevorzugt 55 bis 70 %, bevorzugt 60, 67, 70 oder 80 %, besonders bevorzugt 60 %. Dabei beziehen sich die Prozentangaben innerhalb des Mantelüberzugs auf den Teil Wirkstoff-Lack ohne einen eventuell vorhandenen Farblack.

Der Anteil des Mantelüberzugs ohne evtl. vorhandenen Farblack in der erfindungsgemäßen Darreichungsform beträgt 5 bis 100 %, bevorzugt 5 bis 80 %, besonders bevorzugt 10 bis 50%, weiterhin besonders bevorzugt beispielsweise etwa mehr als 10%, 15%, 20%, 30%, 40% oder 50% bezogen auf das Kerngewicht.

Bezogen auf das Gesamtgewicht der Darreichungsform beträgt der Anteil des Mantelüberzugs ohne evtl. vorhandenen Farblack an der erfindungsgemäßen Darreichungsform 4 bis 50 %, bevorzugt 5 bis 45 %, besonders bevorzugt 9 bis 33%, weiterhin besonders bevorzugt beispielsweise etwa mehr als 10%, 15%, 20%, 25%, 30% oder 33%.

Das Gewicht des Mantelüberzugs in der erfindungsgemäßen Darreichungsform beträgt allgemein 10 bis 300 mg, bevorzugt 20 bis 300 mg, bevorzugt 25 bis 250 mg, besonders bevorzugt 50 bis 200 mg, weiterhin besonders bevorzugt 100 mg bis 150 mg. Das Gewicht des Mantelüberzugs in der erfindungsgemäßen Darreichungsform für den Fall, dass nur ein Wirkstoff in der Wirkstoffschicht ist, beträgt 10 bis 300 mg, bevorzugt 10 bis 250 mg, bevorzugt 10 bis 150 mg, besonders bevorzugt 10 bis 100 mg, weiterhin besonders bevorzugt 10 mg bis 80 mg. Das Gewicht des Mantelüberzugs in der erfindungsgemäßen Darreichungsform für den Fall, dass zwei Wirkstoffe in der Wirkstoffschicht sind, beträgt 10 bis 300 mg, bevorzugt 20 bis 250 mg, bevorzugt 30 bis 200 mg, besonders bevorzugt 40 bis 200 mg, weiterhin besonders bevorzugt 40 mg bis 150 mg. Dabei ist in das Gewicht des Mantelüberzugs nur das des Wirkstoff-Lacks ohne einen eventuell vorhandenen Farblack hineingerechnet.

Die Dicke des Mantelüberzugs beträgt 25 bis 1500 µm, bevorzugt 50 bis 1500 µm, besonders bevorzugt 50 bis 1200 µm, weiterhin bevorzugt 75 bis 1200 µm, ganz besonders bevorzugt 100 bis 1000 µm, weiterhin besonders bevorzugt mehr als 25 µm, 50 µm, 75 µm, 100 µm, 150 µm, 200 µm, 250 µm.

Die erfindungsgemäße Darreichungsform erfüllt die Arzneibuchanforderung an Content Uniformity (beispielsweise gemäß USP 31, Uniformity of dosage units). Dabei liegt der Akzeptanzwert unterhalb von 15 % und die prozentuale Standardabweichung des Gehalts des Angiotensin-II Antagonisten und/oder des Diuretikums im Mantelüberzug unterhalb von 6,25 %, bevorzugt unterhalb von 6 %, besonders bevorzugt unterhalb von 5% bei n=10 Einzeldosen, oder unterhalb von 7,5 %, bevorzugt unterhalb von 6 %, besonders bevorzugt unterhalb von 5% bei n=10 Einzeldosen. Dabei beträgt der Durchschnittsgehalt an Angiotensin-II Antagonisten und/oder Diuretikum im Mantelüberzug, berechnet aus den bestimmten n=10 oder n=30 Einzelgehalten zwischen 95 und 105 %, bevorzugt zwischen 97 und 103 %, besonders bevorzugt zwischen 98,5 und 101,5 % bezogen auf den Sollgehalt der Tabletten.

Die erfindungsgemäße Darreichungsform zeigt bei der Prüfung auf Abrieb (beispielsweise gemäß USP 31 <1216> Tablet Friability) einen sehr geringen Abrieb von weniger als 0,5 %, bevorzugt von weniger als 0,1 %, besonders bevorzugt von weniger als 0,01 % bis zu gar keinen messbaren Abrieb bezogen auf das Gewicht der Darreichungsform.

Die erfindungsgemäße Darreichungsform zeigt bei der Prüfung auf Bruchfestigkeit mit einem geeigneten Tablettentestgerät (beispielsweise Schleuniger Typ 6D oder Typ 8M, Dr. Schleuniger Pharmatron AG, Solothurn, Schweiz) eine Bruchfestigkeit von größer als 200 N, bevorzugt größer als 300 N. In einer besonders bevorzugten Ausführungsform kommt es während der Ausführung der Prüfung auf Bruchfestigkeit bis zu 449 N weder zu einem Brechen noch zum Abbröckeln des Mantelüberzugs, sondern höchstens zu einer leichten plastischen Verformung.

Der Mantelüberzug der erfindungsgemäßen Darreichungsform lässt sich bei der Prüfung auf Zerfallszeit (beispielsweise gemäß USP 31 <701> Disintegration) mit gereinigtem Wasser als Medium bei 37°C vollständig innerhalb von 40 Minuten, bevorzugt innerhalb von 25 Minuten, besonders bevorzugt innerhalb von 10 Minuten vom Kern ablösen.

Die erfindungsgemäße Darreichungsform setzt bei der Prüfung auf in-vitro Dissolution mindestens 85 % des Nifedipins oder Nisoldipins (bezogen auf die deklarierte Gesamtmenge des jeweiligen Wirkstoffes) über einen Zeitraum von mindestens 4 und höchstens 24 Stunden frei, bevorzugt 5 bis 17 % des Nifedipins oder Nisoldipins innerhalb von 4 Stunden und 43 bis 80 %, besonders bevorzugt 45 bis 75 % des Nifedipins oder Nisoldipins innerhalb von 12 Stunden frei. Außerdem setzt die erfindungsgemäße Darreichungsform bei der Prüfung auf in-vitro Dissolution mindestens 60 %, bevorzugt 70 %, besonders bevorzugt mindestens 80 % des Angiotensin-II Antagonisten und/oder des Diuretikums (bezogen auf die deklarierte Gesamtmenge des jeweiligen Wirkstoffes) innerhalb von 30 Minuten frei. Die Prüfung auf in-vitro Dissolution wird gemäß USP-Freisetzungsmethode mit Apparatur 2 (Paddle), bei 50 bis 100 Umdrehungen, bevorzugt 75 oder 100 Umdrehungen, in 900 bis 1000 ml eines geeigneten Mediums bei 37°C durchgeführt. Das geeignete Medium kann je nach Löslichkeit der Wirkstoffe beispielsweise ausgewählt werden aus 0,1 N Salzsäure (pH 1,0), 0,01 N Salzsäure (pH 2,0), Acetatpuffer pH 4,5, Phosphatpuffer pH 4,5, Phosphatpuffer pH 6,8 und Phosphatpuffer pH 8,0 mit einem Zusatz von 0-1 % Natriumlaurylsulfat oder 0-1 % Polysorbat 20 (Tween 20), bevorzugt 0,4-1,0 % Natriumlaurylsulfat. Die Prüfung auf in-vitro Dissolution kann für alle in einer Formulierung enthaltenen Wirkstoffe simultan im gleichen Medium durchgeführt werden oder unabhängig voneinander in unterschiedlichen Medien für die einzelnen Wirkstoffe.

Weiterer Gegenstand der vorliegenden Erfindung ist ein Verfahren zur Herstellung einer erfindungsgemäßen Darreichungsform umfassend ein osmotisches Wirkstofffreisetzungssystem als Kern, und eine Mantelschicht, wobei zum Aufbringen des Mantelüberzugs die Kerne, beispielsweise 800 g, in einen Trommelcoater mit einer Nennkapazität von 1 kg Füllgut gegeben werden und eine wässrige Lacksuspension, beispielsweise 1600 g, enthaltend mindestens einen Angiotensin-II Antagonisten und/oder mindestens ein Diuretikum, mindestens ein Film bildendes Polymer und gegebenenfalls weitere Hilfsstoffe aufgesprüht werden. Die Zulufttemperatur beträgt 40 bis 70°C, bevorzugt 55 bis 65°C, besonders bevorzugt 60°C beispielsweise bei einer Zuluftmenge von ca. 120 m³/h und einer Trommeldrehzahl von 10 bis 18 UpM, bevorzugt 12 bis 15 UpM. Als Sprühdüse kann beispielsweise eine Rundstrahldüse mit einem Durchmesser von 0,8 bis 1,2 mm oder eine Flachstrahldüse bei einem Zerstäuberdruck von 1,6 bis 2,2 bar verwendet werden. Eine anfängliche Sprührate von beispielsweise 4 g/min kann während des Aufsprühvorgangs kontinuierlich oder in diskreten Stufen, zum Beispiel alle 10 bis 30 Minuten in Schritten von z. B. je 1 g/min oder von je 10% der aktuellen Sprührate auf bis zu 18 g/min gesteigert werden. Nach Beendigung des Aufsprühvorgangs, beispielsweise nach 180 bis 240 Minuten, können die Tabletten ohne weiteres Aufsprühen für beispielsweise für 5 bis 60 Minuten, bevorzugt 10 bis 30 Minuten, oder bis zur vollständigen Abkühlung auf Raumtemperatur in der Trommel bei diskontinuierlicher oder kontinuierlicher Trommeldrehung, bevorzugt kontinuierlich mit einer Trommeldrehzahl von 6 bis 15 UpM, poliert werden. Für größere oder kleinere Trommelcoater können die Prozessparameter entsprechend angepasst werden. Geeignete Prozessparameter für unterschiedlich große Trommelcoater sind in den Beispielen 2, 9 und 13 beispielhaft beschrieben.

Bevorzugt ist die Sprühdüse an einem Sprüharm befestigt, der während des Aufsprühvorgangs verstellt werden kann, um ein optimales Aufsprühen des Wirkstoffs zu gewährleisten.

Die wässrige Lacksuspension enthält bevorzugt ca. 20 bis ca. 30 %, besonders bevorzugt 25-30 % Feststoffe bezogen auf das Gesamtgewicht der Lacksuspension.

Der Endpunkt des Aufsprühvorgangs kann auf unterschiedliche Weise bestimmt werden. Einerseits kann die Menge an aufzusprühender Lacksuspension fest vorgegeben werden; dabei ist typischerweise ein Sprühverlust von ca. 5 bis 20 %, bevorzugt 10 - 15 % zu berücksichtigen. Andererseits kann der Endpunkt auch durch Inprozesskontrollen an den zu überziehenden Tabletten bestimmt werden. Zu diesem Zweck werden bevorzugt im Rahmen einer at-line Inprozesskontrolle während des Aufsprühvorgangs zu definierten Zeitpunkten Tabletten entnommen und es wird daran mittels Wägung und/oder spektroskopischer Methoden wie beispielsweise NIR-, Raman- oder Terahertz-Spektroskopie die Schichtdicke oder der Wirkstoffgehalt der Lackschicht ermittelt. Aufgrund der erhaltenen In-Prozess-Kontroll-Werte wird der Aufsprühvorgang genau bei Erreichen der Zielauftragsmenge beendet. Die genannten spektroskopischen Methoden eignen sich grundsätzlich auch für eine in-line-Inprozesskontrolle. In diesem Fall wird eine Spektroskopiesonde in den Trommelcoater eingebaut, so dass fortlaufend während des Aufsprühvorgangs einzelne Tabletten vermessen werden können, ohne dass diese hierzu aus dem Trommelcoater entnommen werden müssen.

Auf die erfindungsgemäße Darreichungsform kann bei Bedarf auf den Mantelüberzug ein weiterer Lack ohne Wirkstoff, beispielsweise ein Lichtschutz- oder Farblack aufgebracht werden. Hierfür können grundsätzlich dieselben Hilfsstoffe eingesetzt werden wie für den Mantelüberzug. Geeignete Materialien hierfür sind insbesondere Polymere wie beispielsweise Polyvinylalkohol, Hydroxypropylcellulose oder Hydroxypropylmethylcellulose, gegebenenfalls in Kombination mit geeigneten Weichmachern wie beispielsweise Polyethylenglykol und Pigmenten wie beispielsweise Titandioxid oder Eisenoxiden.

Bevorzugt sind insbesondere folgende kommerziell erhältliche Präparate, sogenannte "Fertiglacke", die bereits weitere pharmazeutische Hilfsstoffe enthalten und einfach in Wasser aufgelöst werden, wie z.Bsp. Opadry II 85F230009 Orange (Colorcon PVA-basierter Fertiglack), Zusammensetzung: partiell hydrolysierter Polyvinylalkohol, Talkum, Polyethylenglykol (PEG 3350), Titandioxid, rotes Eisenoxid, gelbes Eisenoxid und Polysorbat 80 (Tween 80).

Weiterer Gegenstand der vorliegenden Erfindung sind orale, einmal täglich applizierbare Arzneimittel, enthaltend eine erfindungsgemäße Darreichungsform.

Weiterer Gegenstand der vorliegenden Erfindung ist die Verwendung der erfindungsgemäßen Darreichungsform zur Prophylaxe, Sekundärprophylaxe oder Behandlung von cardiovaskulären Erkrankungen, z.B. Bluthochdruck.

Weiterer Gegenstand der vorliegenden Erfindung ist die Verwendung der erfindungsgemäßen Darreichungsform zur Herstellung eines Arzneimittels zur Prophylaxe, Sekundärprophylaxe oder Behandlung von cardiovaskulären Erkrankungen, beispielsweise Bluthochdruck, Myocardinfarkt, Re-Infarkt, Angina pectoris, koronarer Herzkrankheit, chronischer Herzinsuffizienz, transistorischen ischämischen Attacken oder dem Insult.

Die Kombination von Nifedipin oder Nisoldipin mit einem Angiotension-II Antagonisten und einem Diuretikum ist besonders geeignet für die Behandlung von Patienten, bei denen die Monotherapie oder die duale Kombinationstherapie nicht die erwünschte Blutdrucksenkung bewirkt hat. Die therapieresistenten Patienten gehören oft zu den Patienten, bei denen die adäquate Kontrolle des Blutdrucks besonders wichtig ist. Calciumantagonisten und Diuretika gehören zwar beide zu Medikamenten, die den Blutdruck unabhängig von dem Renin-Angiotensin-System senken, sie unterscheiden sich jedoch im Wirkmechanismus. Die Calciumantagonisten sind primär Vasodilatatoren mit einer schwachen natriuretischen Wirkung, während auf die Diuretika (Thiazide) das Gegenteil zutrifft. Wenn das Renin-Angiotensin-System inhibiert ist, wirken die Diuretika additiv zu den Calciumantagonisten. Überraschenderweise konnte gezeigt werden, dass die oben beschriebene dreifache Kombination auch bei therapieresistenten Patienten zu einer adäquaten Kontrolle des Blutdrucks geführt hat.

Die Erfindung wird nachstehend durch bevorzugte Ausführungsbeispiele näher erläutert, auf welche sie jedoch nicht eingeschränkt ist. Soweit nicht anders angegeben, beziehen sich nachstehend alle Mengenangaben auf Gewichtsprozente.

### Experimenteller Teil

### Beispiel 1: Osmotisches Freisetzungssystem (Zweikammersystem)

Kernzusammensetzung in mg/Kern (deklarierter Gehalt = 30 mg Nifedipin)

### Wirkstoffschicht:

| | |
|---|---|
| Nifedipin, mikronisiert | 33,0 mg |
| Hydroxypropylmethylcellulose (5 cp) | 8,2 mg |
| Polyethylenoxid (Molgewicht 200.000) | 122,2 mg |
| Magnesiumstearat | 0,4 mg |
| Summe | 163,8 mg |

### Osmoseschicht:

| | |
|---|---|
| Hydroxypropylmethylcellulose (5 cp) | 4,1 mg |
| Natriumchlorid | 23,9 mg |
| Polyethylenoxid (Molgewicht 5.000.000) | 52.9 mg |
| Eisenoxid rot | 0,8 mg |
| Magnesiumstearat | 0,2 mg |
| Summe | 81,9 mg |

### Hülle (osmotische Membran)

| | |
|---|---|
| Celluloseacetat | 32,3 mg |
| Polyethylenglykol 3.350 | 1,7 mg |
| Summe | 34,0 mg |

### Herstellung:

Die Komponenten der Wirkstoffschicht wurden gemischt und trocken granuliert. Ebenso wurden die Komponenten der Osmoseschicht gemischt und trocken granuliert. Auf einer Zweischichttablettenpresse wurden beide Granulate zu einer Zweischichttablette verpresst. Die Tabletten wurden mit einer acetonischen Lösung von Celluloseacetat und Polyethylenglykol beschichtet und getrocknet.

Anschließend wurde bei jeder Tablette auf der Wirkstoffseite eine Öffnung von 0.9 mm Durchmesser mittels eines Laserstrahls angebracht.

Die so erhaltenen Kerne nach dem Prozess hatten einen Durchmesser von 8,8 mm, eine Höhe von 4,6 mm und ein Gewicht von 276,6 mg ± 4,8 mg.

### Beispiel la: Osmotisches Freisetzungssystem (Zweikammersystem)

Kernzusammensetzung in mg/Kern (deklarierter Gehalt = 60 mg Nifedipin)

### Wirkstoffschicht:

| | |
|---|---|
| Nifedipin, mikronisiert | 66,0 mg |
| Hydroxypropylmethylcellulose (5 cp) | 16,4 mg |
| Polyethylenoxid (Molgewicht 200.000) | 244,4 mg |
| Magnesiumstearat | 0,8 mg |
| Summe | 327,6 mg |

### Osmoseschicht:

| | |
|---|---|
| Hydroxypropylmethylcellulose (5 cp) | 8,2 mg |
| Natriumchlorid | 47,8 mg |
| Polyethylenoxid (Molgewicht 5.000.000) | 105.8 mg |
| Eisenoxid rot | 1,6 mg |
| Magnesiumstearat | 0,4 mg |
| Summe | 163,8 mg |

### Hülle (osmotische Membran)

| | |
|---|---|
| Celluloseacetat | 38,0 mg |
| Polyethylenglykol 3.350 | 2,0 mg |
| Summe | 40,0 mg |

### Herstellung: analog Beispiel 1

Die so erhaltenen Kerne nach dem Prozess hatten einen Durchmesser von 10,6 mm, eine Höhe von 6,4 mm und ein Gewicht von 531,0 mg ± 3,9 mg.

### Beispiel 1b: Osmotisches Freisetzungssystem (Zweikammersystem)

Kernzusammensetzung in mg/Kern (deklarierter Gehalt = 20 mg Nifedipin)

### Wirkstoffschicht:

| | |
|---|---|
| Nifedipin, mikronisiert | 22,0 mg |
| Hydroxypropylmethylcellulose (5 cp) | 5,5 mg |
| Polyethylenoxid (Molgewicht 200.000) | 81,5 mg |
| Magnesiumstearat | 0,3 mg |
| Summe | 109,3 mg |

### Osmoseschicht:

| | |
|---|---|
| Hydroxypropylmethylcellulose (5 cp) | 3,6 mg |
| Natriumchlorid | 21,2 mg |
| Polyethylenoxid (Molgewicht 5.000.000) | 47,0 mg |
| Eisenoxid rot | 0,7 mg |
| Magnesiumstearat | 0,2 mg |
| Summe | 72,7 mg |

### Hülle (osmotische Membran)

| | |
|---|---|
| Celluloseacetat | 33,2 mg |
| Polyethylenglykol 3.350 | 1,7 mg |
| Summe | 34,9 mg |

### Herstellung: analog Beispiel 1

Die so erhaltenen Kerne nach dem Prozess hatten einen Durchmesser von 8,3 mm, eine Höhe von 4,2 mm und ein Gewicht von 216,0 mg ± 3,9 mg.

### Beispiel 2: Beschichtete Kerne

### Herstellung:

500 g der Fertiglackmischung Colorcon Opadry II 85G25457, enthaltend einen hohen Anteil an roten Eisenoxidpigment-Partikeln, wurden gemäß der Vorschrift des Herstellers rekonstituiert, indem die Fertiglackmischung mittels Propellerrührer in 2000 ml Gereinigtes Wasser eingerührt und dann für weitere 45 Minuten gerührt wurde (Lacksuspension).

810,5 g (entsprechend 2930 Stück) Kerne gemäß Beispiel 1 (deklarierter Gehalt = 30 mg Nifedipin) wurden in einen Coater Glatt GC300 eingebracht und bei einer Zulufttemperatur von 60°C, einer Zuluftmenge von 120 m³/h und einer Trommeldrehzahl von 10 UpM (Umdrehungen pro Minute) vorgewärmt. Die Parameter Zulufttemperatur (60°C), Zuluftmenge (120 m³/h) und Trommeldrehzahl (10 UpM) wurden während des gesamten Coating-Prozesses konstant gehalten.

Für das Aufsprühen der Lacksuspension wurde eine Rundstrahldüse mit einem Durchmesser von 0,8 mm bei einem Zerstäuberdruck 1,6 bar verwendet, wobei der bewegliche Sprüharm während des gesamten Coating-Prozesses jeweils so eingestellt war, dass ein homogenes Sprühbild erzielt wurde.

Die Lacksuspension wurde mit einer über die Dauer des Coating-Prozesses stufenweise gesteigerten Sprührate auf die Kerne aufgetragen, und zwar beginnend mit 6 g/min und endend mit 16 g/min mit folgenden Stufen:

| Zeit [min] | | Sprührate [g/min] | |
|---|---|---|---|
| 0 - 40 | | 6 | |
| 40 - 50 | | 7 | |
| 50 - 70 | | 8 | |
| 70 - 80 | | 9 | |
| 80 - 90 | | 10 | |
| 90 - 100 | | 11 | |
| 100 - 130 | | 12 | |
| 130 - 140 | | 13 | |
| 140 - 150 | | 14 | |
| 150 - 160 | | 15 | |
| 160 - 210 | | 16 | |

Die Gesamtzeit des Aufsprühens betrug 210 Minuten. Danach wurden die Tabletten für weitere 10 Minuten ohne weiteres Aufsprühen in der Trommel poliert.

Vor dem Coating-Prozess, zu verschiedenen Zeitpunkten während des Coating-Prozesses, und sofort nach dem Coating-Prozess wurden Muster der Tabletten entnommen und die Gewichtszunahme bestimmt.

Folgende Ergebnisse wurden erhalten:

| Zeit [min] | | Gewicht [mg] | | Standardabweichung [mg] | |
|---|---|---|---|---|---|
| 0 | | 276,6 | | 4,8 | |
| 30 | | 298,5 | | 4,8 | |
| 60 | | 311,0 | | 5,3 | |
| 90 | | 326,3 | | 6,8 | |
| 120 | | 351,6 | | 9,1 | |
| 150 | | 380,4 | | 8,4 | |
| 180 | | 414,4 | | 10,9 | |
| 210 | | 446,1 | | 12,2 | |

Die so erhaltenen Tabletten hatten demnach ein Gewicht von 446,1 mg ± 12,2 mg. Dies entspricht einem Lackauftrag von 170 mg.

Die erhaltenen Tabletten hatten einen Durchmesser von 10,3 mm und eine Höhe von 6,0 mm. Es wurde ein Querschnitt durch eine Tablette angefertigt und unter einem Auflichtmikroskop betrachtet. Der GITS- Kern, bestehend aus der Zweischichttablette mit einem ca. 0,13 mm dicken Cellulosacetat-Lack, war von einer gleichmäßigen ca. 0,7 mm dicken einheitlich rot gefärbten Schicht umhüllt.

Die erhaltenen Tabletten wurden mittels eines Bruchfestigkeitstesters (Schleuniger Typ 6D, Dr. Schleuniger Pharmatron AG, Solothurn, Schweiz) untersucht. Bei der maximal möglichen Kraft von 449 N kam es nicht zu einem Brechen der Tablette, sondern lediglich zu einer leichten plastischen Verformung des Filmlacks.

Die erhaltenen Tabletten wurden der Prüfung auf Abrieb gemäß USP 31 (<1216> Tablet Friability) unterzogen. Es konnte kein Abrieb festgestellt werden.

Die erhaltenen Tabletten wurden der Prüfung auf Zerfallszeit gemäß USP 31 (<701> Disintegration) mit Gereinigtem Wasser als Medium bei 37°C unterzogen und fortlaufend beobachtet. Nach spätestens 25 Minuten war der Filmlack vollständig abgelöst.

Die erhaltenen Tabletten wurden einem Freisetzungstest gemäß USP 31 (<711> Dissolution) apparatus 2 (paddle apparatus) mit 50 UpM (Umdrehungen pro Minute) und 1000 ml Gereinigtem Wasser als Medium bei 37°C unterzogen und fortlaufend beobachtet. Nach spätestens 25 Minuten war der Filmlack vollständig abgelöst.

### Beispiel 3: Osmotisches Freisetzungssystem mit Telmisartan-haltiger Mantelschicht

In einer Lacksuspension auf Basis eines Polyvinylalkohol-Derivats werden 30 % Telmisartan (bezogen auf den Feststoffanteil der Lacksuspension) homogen suspendiert.

In einen Trommelcoater werden so viele Kerne gemäß Beispiel 1, 1a oder 1b eingebracht, dass dieser mit ca. 75% der typischen Ansatzgröße beladen ist. Auf diese Kerne wird entsprechend Beispiel 2 die Telmisartan-haltige Lacksuspension auflackiert, bis das Gesamtgewicht der Tabletten ca. 125% der typischen Ansatzgröße des Coaters entspricht.

### Beispiel 4: Osmotisches Freisetzungssystem mit Candesartan-haltiger Mantelschicht

In einer Lacksuspension auf Basis eines Polyvinylalkohol-Derivats werden 20 % Candesartan cilexetil (bezogen auf den Feststoffanteil der Lacksuspension) homogen suspendiert.

In einen Trommelcoater werden so viele Kerne gemäß Beispiel 1, 1a oder 1b eingebracht, dass dieser mit ca. 80% der typischen Ansatzgröße beladen ist. Auf diese Kerne wird entsprechend Beispiel 2 die Candesartan cilexetil-haltige Lacksuspension auflackiert, bis das Gesamtgewicht der Tabletten ca. 120% der typischen Ansatzgröße des Coaters entspricht.

### Beispiel 5: beispielhafte Zusammensetzungen von Nifedipin + Telmisartan haltigen Tabletten

Alle Angaben in mg, Herstellung gemäß Beispiel 1, 1a, 1b, 2 und 3 unter Anpassung der Mengen und Konzentrationen.

| Formulierung | 5a | 5b | 5c | 5d | 5e | 5f | 5g | 5h | 5i | 5j | 5k | 5l |
|---|---|---|---|---|---|---|---|---|---|---|---|---|
| Wirkstoffschicht | | | | | | | | | | | | |
| Nifedipin, mikronisiert | 33,0 | 33,0 | 33,0 | 33,0 | 33,0 | 33,0 | 33,0 | 33,0 | 22,0 | 22,0 | 66,0 | 66,0 |
| HMPC (5 cp) | 8,2 | 8,2 | 8,2 | 8,2 | 8,2 | 8,2 | 8,2 | 8,2 | 5,5 | 5,5 | 16,4 | 16,4 |
| PEO 200.000 | 122,2 | 122,2 | 122,2 | 122,2 | 122,2 | 122,2 | 122,2 | 122,2 | 81,5 | 81,5 | 244,4 | 244,4 |
| Magnesiumstearat | 0,4 | 0,4 | 0,4 | 0,4 | 0,4 | 0,4 | 0,4 | 0,4 | 0,3 | 0,3 | 0,8 | 0,8 |

| Osmoseschicht | | | | | | | | | | | | |
|---|---|---|---|---|---|---|---|---|---|---|---|---|
| HMPC (5 cp) | 4,1 | 4,1 | 4,1 | 4,1 | 4,1 | 4,1 | 4,1 | 4,1 | 3,6 | 3,6 | 8,2 | 8,2 |
| Natriumchlorid | 23,9 | 23,9 | 23,9 | 23,9 | 23,9 | 23,9 | 23,9 | 23,9 | 21,2 | 21,2 | 47,8 | 47,8 |
| PEO 5.000.000 | 52,9 | 52,9 | 52,9 | 52,9 | 52,9 | 52,9 | 52,9 | 52,9 | 47,0 | 47,0 | 105,8 | 105,8 |
| Eisenoxid rot | 0,8 | 0,8 | 0,8 | 0,8 | 0,8 | 0,8 | 0,8 | 0,8 | 0,7 | 0,7 | 1,6 | 1,6 |
| Magnesiumstearat | 0,2 | 0,2 | 0,2 | 0,2 | 0,2 | 0,2 | 0,2 | 0,2 | 0,2 | 0,2 | 0,4 | 0,4 |

| osmotische Membran | | | | | | | | | | | | |
|---|---|---|---|---|---|---|---|---|---|---|---|---|
| Celluloseacetat | 32,3 | 32,3 | 32,3 | 32,3 | 32,3 | 32,3 | 32,3 | 32,3 | 33,2 | 33,2 | 38,0 | 38,0 |
| PEG 3.350 | 1,7 | 1,7 | 1,7 | 1,7 | 1,7 | 1,7 | 1,7 | 1,7 | 1,7 | 1,7 | 2,0 | 2,0 |

| Wirkstoff-Lack | | | | | | | | | | | | |
|---|---|---|---|---|---|---|---|---|---|---|---|---|
| Telmisartan, mikronisiert | 20,0 | 20,0 | 20,0 | 20,0 | 80,0 | 80,0 | 80,0 | 80,0 | 20,0 | 80,0 | 20,0 | 80,0 |
| Eisenoxid rot | 1,0 | 1,0 | 1,0 | 1,0 | 1,0 | 1,0 | 1,0 | 1,0 | 1,0 | 1,0 | 1,0 | 1,0 |
| Kollicoat IR | 53,5 | 51,4 | | | 116,0 | 99,1 | | | | | | |
| Kollidon VA 64 | 7,5 | 5,7 | | | 16,0 | 11,0 | | | | | | |
| Titandioxid | | 11,8 | | | | 22,7 | | | | | | |
| Kaolin | 12,0 | 13,4 | | | 26,0 | 25,9 | | | | | | |
| Natriumlaurylsulfat | 2,0 | 1,7 | | | 4,0 | 3,3 | | | | | | |
| Sepifilm IR colorless | | | 84,0 | | | | 162,0 | | | | | |
| Opadry II 85F28393 | | | | 84,0 | | | | 162,0 | 84,0 | 121,5 | 84,0 | 189,0 |

### Beispiel 6: beispielhafte Zusammensetzungen von Nifedipin + Candesartan cilexetil haltigen Tabletten

Alle Angaben in mg, Herstellung gemäß Beispiel 1, 1a, 1b, 2 und 4 unter Anpassung der Mengen und Konzentrationen.

| Formulierung | 6a | 6b | 6c | 6d | 6e | 6f | 6g | 6h | 6i | 6j | 6k | 6l |
|---|---|---|---|---|---|---|---|---|---|---|---|---|
| Wirkstoffschicht | | | | | | | | | | | | |
| Nifedipin, mikronisiert | 33,0 | 33,0 | 33,0 | 33,0 | 33,0 | 33,0 | 33,0 | 33,0 | 22,0 | 22,0 | 66,0 | 66,0 |
| HMPC (5 cp) | 8,2 | 8,2 | 8,2 | 8,2 | 8,2 | 8,2 | 8,2 | 8,2 | 5,5 | 5,5 | 16,4 | 16,4 |
| PEO 200.000 | 122,2 | 122,2 | 122,2 | 122,2 | 122,2 | 122,2 | 122,2 | 122,2 | 81,5 | 81,5 | 244,4 | 244,4 |
| Magnesiumstearat | 0,4 | 0,4 | 0,4 | 0,4 | 0,4 | 0,4 | 0,4 | 0,4 | 0,3 | 0,3 | 0,8 | 0,8 |

| Osmoseschicht | | | | | | | | | | | | |
|---|---|---|---|---|---|---|---|---|---|---|---|---|
| HMPC (5 cp) | 4,1 | 4,1 | 4,1 | 4,1 | 4,1 | 4,1 | 4,1 | 4,1 | 3,6 | 3,6 | 8,2 | 8,2 |
| Natriumchlorid | 23,9 | 23,9 | 23,9 | 23,9 | 23,9 | 23,9 | 23,9 | 23,9 | 21,2 | 21,2 | 47,8 | 47,8 |
| PEO 5.000.000 | 52,9 | 52,9 | 52,9 | 52,9 | 52,9 | 52,9 | 52,9 | 52,9 | 47,0 | 47,0 | 105,8 | 105,8 |
| Eisenoxid rot | 0,8 | 0,8 | 0,8 | 0,8 | 0,8 | 0,8 | 0,8 | 0,8 | 0,7 | 0,7 | 1,6 | 1,6 |
| Magnesiumstearat | 0,2 | 0,2 | 0,2 | 0,2 | 0,2 | 0,2 | 0,2 | 0,2 | 0,2 | 0,2 | 0,4 | 0,4 |

| osmotische Membran | | | | | | | | | | | | |
|---|---|---|---|---|---|---|---|---|---|---|---|---|
| Celluloseacetat | 32,3 | 32,3 | 32,3 | 32,3 | 32,3 | 32,3 | 32,3 | 32,3 | 33,2 | 33,2 | 38,0 | 38,0 |
| PEG 3.350 | 1,7 | 1,7 | 1,7 | 1,7 | 1,7 | 1,7 | 1,7 | 1,7 | 1,7 | 1,7 | 2,0 | 2,0 |

| Wirkstoff-Lack | | | | | | | | | | | | |
|---|---|---|---|---|---|---|---|---|---|---|---|---|
| Candesartan cilexetil, mikronisiert | 4,0 | 4,0 | 4,0 | 4,0 | 32,0 | 32,0 | 32,0 | 32,0 | 4,0 | 32,0 | 4,0 | 32,0 |
| Eisenoxid rot | 1,0 | 1,0 | 1,0 | 1,0 | 1,0 | 1,0 | 1,0 | 1,0 | 1,0 | 1,0 | 1,0 | 1,0 |
| Kollicoat IR | 10,7 | 12,2 | | | 85,6 | 80,8 | | | | | | |
| Kollidon VA 64 | 1,5 | 1,4 | | | 12,0 | 9,0 | | | | | | |
| Titandioxid | | 2,8 | | | | 18,5 | | | | | | |
| Kaolin | 2,4 | 3,2 | | | 19,2 | 21,1 | | | | | | |
| Natrium-laurylsulfat | 0,4 | 0,4 | | | 3,2 | 2,6 | | | | | | |
| Sepifilm IR colorless | | | 20,0 | | | | 132,0 | | | | | |
| Opadry II 85F28393 | | | | 20,0 | | | | 132,0 | 20,0 | 99,0 | 20,0 | 132,0 |

### Beispiel 7: beispielhafte Zusammensetzungen von Nifedipin + Telmisartan + HCT haltigen Tabletten

Alle Angaben in mg, Herstellung gemäß Beispiel 1, 1a, 1b, 2 und 3 unter Anpassung der Mengen und Konzentrationen.

| Formulierung | 7a | 7b | 7c | 7d | 7e | 7f | 7g | 7h | 7i | 7j | 7k | 7l |
|---|---|---|---|---|---|---|---|---|---|---|---|---|
| Wirkstoffschicht | | | | | | | | | | | | |
| Nifedipin, mikronisiert | 33,0 | 33,0 | 33,0 | 33,0 | 33,0 | 33,0 | 33,0 | 33,0 | 22,0 | 22,0 | 66,0 | 66,0 |
| HMPC (5 cp) | 8,2 | 8,2 | 8,2 | 8,2 | 8,2 | 8,2 | 8,2 | 8,2 | 5,5 | 5,5 | 16,4 | 16,4 |
| PEO 200.000 | 122,2 | 122,2 | 122,2 | 122,2 | 122,2 | 122,2 | 122,2 | 122,2 | 81,5 | 81,5 | 244,4 | 244,4 |
| Magnesiumstearat | 0,4 | 0,4 | 0,4 | 0,4 | 0,4 | 0,4 | 0,4 | 0,4 | 0,3 | 0,3 | 0,8 | 0,8 |

| Osmoseschicht | | | | | | | | | | | | |
|---|---|---|---|---|---|---|---|---|---|---|---|---|
| HMPC (5 cp) | 4,1 | 4,1 | 4,1 | 4,1 | 4,1 | 4,1 | 4,1 | 4,1 | 3,6 | 3,6 | 8,2 | 8,2 |
| Natriumchlorid | 23,9 | 23,9 | 23,9 | 23,9 | 23,9 | 23,9 | 23,9 | 23,9 | 21,2 | 21,2 | 47,8 | 47,8 |
| PEO 5.000.000 | 52,9 | 52,9 | 52,9 | 52,9 | 52,9 | 52,9 | 52,9 | 52,9 | 47,0 | 47,0 | 105,8 | 105,8 |
| Eisenoxid rot | 0,8 | 0,8 | 0,8 | 0,8 | 0,8 | 0,8 | 0,8 | 0,8 | 0,7 | 0,7 | 1,6 | 1,6 |
| Magnesiumstearat | 0,2 | 0,2 | 0,2 | 0,2 | 0,2 | 0,2 | 0,2 | 0,2 | 0,2 | 0,2 | 0,4 | 0,4 |

| osmotische Membran | | | | | | | | | | | | |
|---|---|---|---|---|---|---|---|---|---|---|---|---|
| Celluloseacetat | 32,3 | 32,3 | 32,3 | 32,3 | 32,3 | 32,3 | 32,3 | 32,3 | 33,2 | 33,2 | 38,0 | 38,0 |
| PEG 3.350 | 1,7 | 1,7 | 1,7 | 1,7 | 1,7 | 1,7 | 1,7 | 1,7 | 1,7 | 1,7 | 2,0 | 2,0 |

| Wirkstoff-Lack | | | | | | | | | | | | |
|---|---|---|---|---|---|---|---|---|---|---|---|---|
| Telmisartan, mikronisiert | 40,0 | 40,0 | 40,0 | 40,0 | 80,0 | 80,0 | 80,0 | 80,0 | 40,0 | 80,0 | 40,0 | 80,0 |
| Hydochlorothiazid, mikronisiert | 12,5 | 12,5 | 12,5 | 12,5 | 25,0 | 25,0 | 25,0 | 25,0 | 12,5 | 12,5 | 12,5 | 25,0 |
| Eisenoxid rot | 1,0 | 1,0 | 1,0 | 1,0 | 1,0 | 1,0 | 1,0 | 1,0 | 1,0 | 1,0 | 1,0 | 1,0 |
| Kollicoat IR | 116,0 | 99,1 | | | 116,0 | 99,1 | | | | | | |
| Kollidon VA 64 | 16,0 | 11,0 | | | 16,0 | 11,0 | | | | | | |
| Titandioxid | | 22,7 | | | | 22,7 | | | | | | |
| Kaolin | 26,0 | 25,9 | | | 26,0 | 25,9 | | | | | | |
| Natrium-laurylsulfat | 4,0 | 3,3 | | | 4,0 | 3,3 | | | | | | |
| Sepifilm IR colorless | | | 162,0 | | | | 162,0 | | | | | |
| Opadry II 85F28393 | | | | 162,0 | | | | 162,0 | 121,5 | 121,5 | 189,0 | 189,0 |

### Beispiel 8: beispielhafte Zusammensetzungen von Nifedipin + Candesartan cilexetil + HCT haltigen Tabletten

Alle Angaben in mg, Herstellung gemäß Beispiel 1, 1a, 1b, 2 und 4 unter Anpassung der Mengen und Konzentrationen.

| Formulierung | 8a | 8b | 8c | 8d | 8e | 8f | 8g | 8h | 8i | 8j | 8k | 8l |
|---|---|---|---|---|---|---|---|---|---|---|---|---|
| Wirkstoffschicht | | | | | | | | | | | | |
| Nifedipin, mikronisiert | 33,0 | 33,0 | 33,0 | 33,0 | 33,0 | 33,0 | 33,0 | 33,0 | 22,0 | 22,0 | 66,0 | 66,0 |
| HMPC (5 cp) | 8,2 | 8,2 | 8,2 | 8,2 | 8,2 | 8,2 | 8,2 | 8,2 | 5,5 | 5,5 | 16,4 | 16,4 |
| PEO 200.000 | 122,2 | 122,2 | 122,2 | 122,2 | 122,2 | 122,2 | 122,2 | 122,2 | 81,5 | 81,5 | 244,4 | 244,4 |
| Magnesiumstearat | 0,4 | 0,4 | 0,4 | 0,4 | 0,4 | 0,4 | 0,4 | 0,4 | 0,3 | 0,3 | 0,8 | 0,8 |
| Osmoseschicht | | | | | | | | | | | | |
| HMPC (5 cp) | 4,1 | 4,1 | 4,1 | 4,1 | 4,1 | 4,1 | 4,1 | 4,1 | 3,6 | 3,6 | 8,2 | 8,2 |
| Natriumchlorid | 23,9 | 23,9 | 23,9 | 23,9 | 23,9 | 23,9 | 23,9 | 23,9 | 21,2 | 21,2 | 47,8 | 47,8 |
| PEO 5.000.000 | 52,9 | 52,9 | 52,9 | 52,9 | 52,9 | 52,9 | 52,9 | 52,9 | 47,0 | 47,0 | 105,8 | 105,8 |
| Eisenoxid- rot | 0,8 | 0,8 | 0,8 | 0,8 | 0,8 | 0,8 | 0,8 | 0,8 | 0,7 | 0,7 | 1,6 | 1,6 |
| Magnesiumstearat | 0,2 | 0,2 | 0,2 | 0,2 | 0,2 | 0,2 | 0,2 | 0,2 | 0,2 | 0,2 | 0,4 | 0,4 |
| osmotische Membran | | | | | | | | | | | | |
| Celluloseacetat | 32,3 | 32,3 | | 32,3 | 32,3 | 32,3 | 32,3 | 32,3 | 33,2 | 33,2 | 38,0 | 38,0 |
| PEG 3.350 | 1,7 | 1,7 | | 1,7 | 1,7 | 1,7 | 1,7 | 1,7 | 1,7 | 1,7 | 2,0 | 2,0 |
| Wirkstoff-Lack | | | | | | | | | | | | |
| Candesartan cilexetil, mikronisiert | 8,0 | 8,0 | 8,0 | 8,0 | 16,0 | 16,0 | 16,0 | 16,0 | 8,0 | 16,0 | 8,0 | 16,0 |
| Hydochlorothiazid, mikronisiert | 12,5 | 12,5 | 12,5 | 12,5 | 12,5 | 12,5 | 12,5 | 12,5 | 12,5 | 12,5 | 12,5 | 12,5 |
| Eisenoxid- rot | 1,0 | 1,0 | 1,0 | 1,0 | 1,0 | 1,0 | 1,0 | 1,0 | 1,0 | 1,0 | 1,0 | 1,0 |
| Kollicoat IR | 85,6 | 80,8 | | | 85,6 | 80,8 | | | | | | |
| Kollidon VA 64 | 12,0 | 9,0 | | | 12,0 | 9,0 | | | | | | |
| Titandioxid | | 18,5 | | | | 18,5 | | | | | | |
| Kaolin | 19,2 | 21,1 | | | 19,2 | 21,1 | | | | | | |
| Natriumlaurylsulfat | 3,2 | 2,6 | | | 3,2 | 2,6 | | | | | | |
| Sepifilm IR colorless | | | 132,0 | | | | 132,0 | | | | | |
| Opadry II 85F28393 | | | | 132,0 | | | | 132,0 | 99,0 | 99,0 | 132,0 | 132,0 |

### Beispiel 9: Herstellung osmotischer Freisetzungssysteme enthaltend 60 mg Nifedipin mit einer Mantelschicht enthaltend 32 mg Candesartan cilexetil und einem Farblack

Zur Herstellung der Lacksuspension wurden 240 g mikronisiertes Candesartan cilexetil (mit einer Partikelgrößenverteilung beschrieben durch X₅₀ < 3 µm und X₉₀ < 7 µm) in 1440 g gereinigtem Wasser mit einem Propellerrührer suspendiert. Nach Zugabe von 360 g des Fertiglacks Opadry II 85F19250 Clear und weiterem Rühren für ca. 45 Minuten wurde eine homogene Suspension erhalten.

In einen Trommelcoater BFC 5 der Firma L. B. BOHLE Maschinen + Verfahren GmbH, D-59320 Ennigerloh, ausgestattet mit der ungeteilten kleinen Trommel und einem Sprüharm mit 2 Sprühdüsen, wurden 3 kg Kerne gemäß Beispiel 1a eingebracht. und bei einer Zulufttemperatur von 60°C, einer Zuluftmenge von 160 m³/h und einer Trommeldrehzahl von 18 UpM (Umdrehungen pro Minute) vorgewärmt. Die Parameter Zulufttemperatur (60°C), Zuluftmenge (160 m³/h) und Trommeldrehzahl (18 UpM) wurden während des gesamten Coating-Prozesses konstant gehalten.

Für das Aufsprühen der Lacksuspension wurden zwei Düsen mit einem Durchmesser von 1,0 mm verwendet, wobei durch einen Sprühdruck von 0,8 bar und einen Formierdruck von 0,7 bar elipsoide Sprühkegel erzielt wurden. Der Sprüharm war während des gesamten Coating-Prozesses so eingestellt, dass ein homogenes Sprühbild erzielt wurde.

Die Lacksuspension wurde mit einer über die Dauer des Coating-Prozesses stufenweise gesteigerten Sprührate auf die Kerne aufgetragen, und zwar innerhalb der ersten Stunde mit 8 g/min und ab der zweiten Stunde mit 12 g/min.

Die Gesamtzeit des Aufsprühens betrug ca. 150 Minuten. Danach wurden die Tabletten für weitere 30 Minuten ohne weiteres Aufsprühen in der Trommel bei einer Trommeldrehzahl von 12 UpM poliert.

Vor dem Coating-Prozess, zu verschiedenen Zeitpunkten während des Coating-Prozesses, und sofort nach dem Coating-Prozess wurden Muster der Tabletten entnommen und die Gewichtszunahme bestimmt. Anhand der Gewichtszunahme wurde der Endpunkt des Aufsprühvorgangs bestimmt.

Folgende Ergebnisse wurden erhalten:

| Zeit [min] | Gewicht [mg] | Standardabweichung [mg] |
|---|---|---|
| 0 | 531,0 | 3,9 |
| 30 | 544,8 | 4,1 |
| 60 | 558,3 | 4,6 |
| 90 | 572,9 | 3,4 |
| 120 | 593,2 | 6,4 |
| 150 | 612,6 | 6,5 |

Die so erhaltenen Tabletten hatten demnach ein Gewicht von 612,6 mg ± 6,5 mg. Dies entspricht einem theoretischen Lackauftrag ca. 81,6 mg. Die erhaltenen Tabletten hatten einen Durchmesser von 10,9 mm und eine Höhe von 7,0 mm. Die erhaltenen Tabletten hatten eine glatte, leicht glänzende Oberfläche.

Die Bruchfestigkeit der erhaltenen Tabletten wurden mittels eines Bruchfestigkeitstesters (Schleuniger Typ 6D, Dr. Schleuniger Pharmatron AG, Solothurn, Schweiz) untersucht. Erst bei Kräften über 400 N kam es zu einem Brechen der Tabletten.

Die erhaltenen Tabletten wurden der Prüfung auf Abrieb gemäß USP 31 (<1216> Tablet Friability) unterzogen. Es konnte kein Abrieb festgestellt werden.

Die erhaltenen Tabletten wurden der Prüfung auf Zerfallszeit gemäß USP 31 (<701> Disintegration) mit Gereinigtem Wasser als Medium bei 37°C unterzogen und fortlaufend beobachtet. Nach spätestens 10 Minuten war der Filmlack vollständig abgelöst.

Die erhaltenen Tabletten wurden einem Freisetzungstest gemäß USP 31 (<711> Dissolution) apparatus 2 (paddle apparatus) mit 75 UpM (Umdrehungen pro Minute) und 900 ml Acetatpuffer pH 4,5 mit einem Zusatz von 0,6 % Natriumlaurylsulfat als Medium bei 37°C unterzogen. Die Wirkstoffgehalte im Freisetzungsmedium wurden mittels HPLC mit UV-Detektion bestimmt. Nach 30 Minuten waren mindestens 80% des Candesartan cilexetils freigesetzt (niedrigster Wert von 12 Einzelwerten). Die Freisetzung von Nifedipin betrug 10% (Mittelwert aus 6 Einzelwerten; Spanne der Einzelwerte: 7 - 12 %) nach 4 Stunden, 52 % (45 - 56 %) nach 12 Stunden und 98 % (91 - 100 %) nach 24 Stunden.

Die erhaltenen Tabletten wurden einem weiteren Freisetzungstest gemäß USP 31 (<711> Dissolution) apparatus 2 (paddle apparatus) mit 75 UpM (Umdrehungen pro Minute) und 900 ml 0,1 N Salzsäure (pH 1,0) mit einem Zusatz von 0,6 % Natriumlaurylsulfat als Medium bei 37°C unterzogen. Die Wirkstoffgehalte im Freisetzungsmedium wurden mittels HPLC mit UV-Detektion bestimmt. Nach 30 Minuten waren Tabletten mindestens 88% des Candesartan cilexetils freigesetzt (niedrigster Wert von 6 Einzelwerten).

Von 10 zufällig ausgewählten Tabletten den erhaltenen Tabletten wurde jeweils der Einzelgehalt an Candesartan cilexetil mittels HPLC mit UV-Detektion bestimmt. Der Mittelwert der Einzelgehalt betrug 32,0 mg bei einer Standardabweichung von 5,0% bezogen auf den Mittelwert. Daraus ergibt sich bei der Prüfung auf Gehaltseinheitlichkeit (content uniformity) ein Akzeptanzwert von 12,1 %.

Zum Auftrag eines zusätzlichen Farblacks wurden 3,065 kg der Tabletten in einen Trommelcoater BFC 5 der Firma L. B. BOHLE Maschinen + Verfahren GmbH, D-59320 Ennigerloh eingebracht und bei einer Zulufttemperatur von 60°C, einer Zuluftmenge von 160 m³/h und einer Trommeldrehzahl von 18 UpM (Umdrehungen pro Minute) vorgewärmt. Die Parameter Zulufttemperatur (60°C), Zuluftmenge (160 m³/h) und Trommeldrehzahl (18 UpM) wurden während des gesamten Coating-Prozesses konstant gehalten. Für das Aufsprühen der Lacksuspension wurden zwei Düsen mit einem Durchmesser von 1,0 mm verwendet, wobei durch einen Sprühdruck von 0,8 bar und einen Formierdruck von 0,7 bar elipsoide Sprühkegel erzielt wurden. Der Sprüharm war während des gesamten Coating-Prozesses so eingestellt, dass ein homogenes Sprühbild erzielt wurde.

Die Lacksuspension wurde durch Dispergieren von 165 g des Fertiglacks Opadry II 85F230009 in 495 g gereinigtem Wasser hergestellt. 444 g der so erhaltenen Farblacksuspension wurden mit einer Sprührate von 8 g/min auf die Tabletten aufgetragen. Die Gesamtzeit des Aufsprühens betrug ca. 55 Minuten. Der Endpunkt des Aufsprühvorgangs wurde durch die vorgegebene Menge an aufzusprühender Lacksuspension bestimmt. Danach wurden die Tabletten für weitere 15 Minuten ohne weiteres Aufsprühen in der Trommel poliert.

Die so erhaltenen lackierten Tabletten hatten ein Gewicht von 635,7 mg ± 6,7 mg. Dies entspricht einem theoretischen Lackauftrag ca. 23,1 mg. Die erhaltenen lackierten Tabletten hatten einen Durchmesser von 11,0 mm und eine Höhe von 7,1 mm. Die erhaltenen lackierten Tabletten hatten eine glatte, leicht glänzende Oberfläche.

Die Wirkstoff-Freisetzung aus den lackierten Tabletten ist gegenüber der Freisetzung aus den Tabletten ohne Farblack um weniger als 5 Minuten verzögert.

### Beispiel 10: Zusammensetzungen von Nifedipin + Candesartan cilexetil-haltigen Tabletten

Alle Angaben in mg, Herstellung gemäß Beispiel 1, 1a, 1b und 9 unter Anpassung der Mengen und Konzentrationen.

| Formulierung | 10a | 10b | 10c | 10d | 10e | 10f | 10g | 10h | 10i | 10j | 10k | 10l |
|---|---|---|---|---|---|---|---|---|---|---|---|---|
| Wirkstoffschicht | | | | | | | | | | | | |
| Nifedipin, mikronisiert | 66,0 | 66,0 | 66,0 | 66,0 | 33,0 | 33,0 | 33,0 | 33,0 | 22,0 | 22,0 | 22,0 | 22,0 |
| HMPC (5 cp) | 16,4 | 16,4 | 16,4 | 16,4 | 8,2 | 8,2 | 8,2 | 8,2 | 5,5 | 5,5 | 5,5 | 5,5 |
| PEO 200.000 | 244,4 | 244,4 | 244,4 | 244,4 | 122,2 | 122,2 | 122,2 | 122,2 | 81,5 | 81,5 | 81,5 | 81,5 |
| Magnesiumstearat | 0,8 | 0,8 | 0,8 | 0,8 | 0,4 | 0,4 | 0,4 | 0,4 | 0,3 | 0,3 | 0,3 | 0,3 |
| Osmoseschicht | | | | | | | | | | | | |
| HMPC (5 cp) | 8,2 | 8,2 | 8,2 | 8,2 | 4,1 | 4,1 | 4,1 | 4,1 | 3,6 | 3,6 | 3,6 | 3,6 |
| Natriumchlorid | 47,8 | 47,8 | 47,8 | 47,8 | 23,9 | 23,9 | 23,9 | 23,9 | 21,2 | 21,2 | 21,2 | 21,2 |
| PEO 5.000.000 | 105,8 | 105,8 | 105,8 | 105,8 | 52,9 | 52,9 | 52,9 | 52,9 | 47,0 | 47,0 | 47,0 | 47,0 |
| Eisenoxid rot | 1,6 | 1,6 | 1,6 | 1,6 | 0,8 | 0,8 | 0,8 | 0,8 | 0,7 | 0,7 | 0,7 | 0,7 |
| Magnesiumstearat | 0,4 | 0,4 | 0,4 | 0,4 | 0,2 | 0,2 | 0,2 | 0,2 | 0,2 | 0,2 | 0,2 | 0,2 |
| osmotische Membran | | | | | | | | | | | | |
| Celluloseacetat | 38,0 | 38,0 | 38,0 | 38,0 | 32,3 | 32,3 | 32,3 | 32,3 | 33,2 | 33,2 | 33,2 | 33,2 |
| PEG 3.350 | 2,0 | 2,0 | 2,0 | 2,0 | 1,7 | 1,7 | 1,7 | 1,7 | 1,7 | 1,7 | 1,7 | 1,7 |
| Wirkstoff-Lack | | | | | | | | | | | | |
| Candesartan cilexetil, mikronisiert | 32,0 | 16,0 | 8,0 | 4,0 | 32,0 | 16,0 | 8,0 | 4,0 | 32,0 | 16,0 | 8,0 | 4,0 |
| Opadry II 85F19250 | 48,0 | 24,0 | 12,0 | 16,0* | 48,0 | 24,0 | 12,0 | 16,0* | 48,0 | 24,0 | 12,0 | 16,0* |
| Farb-Lack | | | | | | | | | | | | |
| Opadry II 85F230009 | 20,0 | 20,0 | 20,0 | 20,0 | 16,0 | 16,0 | 16,0 | 16,0 | 14,0 | 14,0 | 14,0 | 14,0 |

| | | | | | | | | | | | | |
|---|---|---|---|---|---|---|---|---|---|---|---|---|
| * bei ansonsten gleicher Zusammensetzung kann die Menge an Fertiglack zwischen 6,0 und 16,0 mg variiert werden. | | | | | | | | | | | | |

### Beispiel 11: beispielhafte Zusammensetzungen von Tabletten enthaltend Nifedipin + Candesartan-cilexetil + ein Diuretikum, ausgewählt aus Chlorthalidon und Hydochlorothiazid, wobei die Wirkstoffe gemeinsam in einem Mantelüberzug vorliegen

Alle Angaben in mg, Herstellung gemäß Beispiel 1,1a, 1b und 9 unter Anpassung der Mengen und Konzentrationen.

| Formulierung | 11a | 11b | 11c | 11d | 11e | 11f | 11g | 11h | 11i | 11J | 11k | 11l |
|---|---|---|---|---|---|---|---|---|---|---|---|---|
| Wirkstoffschicht | | | | | | | | | | | | |
| Nifedipin, mikronisiert | 33,0 | 33,0 | 33,0 | 33,0 | 33,0 | 33,0 | 33,0 | 33,0 | 22,0 | 22,0 | 66,0 | 66,0 |
| HMPC (5 cp) | 8,2 | 8,2 | 8,2 | 8,2 | 8,2 | 8,2 | 8,2 | 8,2 | 5,5 | 5,5 | 16,4 | 16,4 |
| PEO 200.000 | 122,2 | 122,2 | 122,2 | 122,2 | 122,2 | 122,2 | 122,2 | 122,2 | 81,5 | 81,5 | 244,4 | 244,4 |
| Magnesiumstearat | 0,4 | 0,4 | 0,4 | 0,4 | 0,4 | 0,4 | 0,4 | 0,4 | 0,3 | 0,3 | 0,8 | 0,8 |
| Osmoseschicht | | | | | | | | | | | | |
| HMPC (5 cp) | 4,1 | 4,1 | 4,1 | 4,1 | 4,1 | 4,1 | 4,1 | 4,1 | 3,6 | 3,6 | 8,2 | 8,2 |
| Natriumchlorid | 23,9 | 23,9 | 23,9 | 23,9 | 23,9 | 23,9 | 23,9 | 23,9 | 21,2 | 21,2 | 47,8 | 47,8 |
| PEO 5.000.000 | 52,9 | 52,9 | 52,9 | 52,9 | 52,9 | 52,9 | 52,9 | 52,9 | 47,0 | 47,0 | 105,8 | 105,8 |
| Eisenoxid rot | 0,8 | 0,8 | 0,8 | 0,8 | 0,8 | 0,8 | 0,8 | 0,8 | 0,7 | 0,7 | 1,6 | 1,6 |
| Magnesiumstearat | 0,2 | 0,2 | 0,2 | 0,2 | 0,2 | 0,2 | 0,2 | 0,2 | 0,2 | 0,2 | 0,4 | 0,4 |
| osmotische Membran | | | | | | | | | | | | |
| Celluloseacetat | 4,1 | 4,1 | 4,1 | 4,1 | 4,1 | 4,1 | 4,1 | 4,1 | 33,2 | 33,2 | 38,0 | 38,0 |
| PEG 3.350 | 23,9 | 23,9 | 23,9 | 23,9 | 23,9 | 23,9 | 23,9 | 23,9 | 1,7 | 1,7 | 2,0 | 2,0 |
| Wirkstoff-Lack | | | | | | | | | | | | |
| Candesartan cilexetil, mikronisiert | 8,0 | 8,0 | 16,0 | 32,0 | 8,0 | 8,0 | 16,0 | 32,0 | 4,0 | 4,0 | 32,0 | 32,0 |
| Hydochlorothiazid, mikronisiert | 12,5 | 25,0 | 25,0 | 25,0 | | | | | 12,5 | | 25,0 | |
| Chlorthalidon, mikronisiert | | | | | 12,5 | 25,0 | 25,0 | 50,0 | | 12,5 | | 50,0 |
| Opadry II 85F19250 | 30,75 | 49,5 | 61,5 | 85,5 | 30,75 | 49,5 | 61,5 | 123,0 | 24,75 | 24,75 | 85,5 | 123,0 |
| Farb-Lack | | | | | | | | | | | | |
| Opadry II 85F230009 | 16,0 | 16,0 | 16,0 | 16,0 | 16,0 | 16,0 | 16,0 | 16,0 | 14,0 | 14,0 | 20,0 | 20,0 |

### Beispiel 12: beispielhafte Zusammensetzungen von Tabletten enthaltend Nifedipin + ggf. Candesartan-cilexetil + ein Diuretikum, ausgewählt aus Chlorthalidon und Hydochlorothiazid, wobei die Wirkstoffe in dem Mantelüberzug in voneinander getrennten, nacheinander aufgetragenen Schichten vorliegen

Alle Angaben in mg, Herstellung gemäß Beispiel 1, 1a, 1b und 9 unter Anpassung der Mengen und Konzentrationen.

| Formulierung | 12a | 12b | 12c | 12d | 12e | 12f | 12g | 12h | 12i | 12j | 12k | 12l |
|---|---|---|---|---|---|---|---|---|---|---|---|---|
| Wirkstoffschicht | | | | | | | | | | | | |
| Nifedipin, mikronisiert | 33,0 | 33,0 | 33,0 | 33,0 | 33,0 | 33,0 | 33,0 | 33,0 | 22,0 | 22,0 | 66,0 | 66,0 |
| HMPC (5 cp) | 8,2 | 8,2 | 8,2 | 8,2 | 8,2 | 8,2 | 8,2 | 8,2 | 5,5 | 5,5 | 16,4 | 16,4 |
| PEO 200.000 | 122,2 | 122,2 | 122,2 | 122,2 | 122,2 | 122,2 | 122,2 | 122,2 | 81,5 | 81,5 | 244,4 | 244,4 |
| Magnesiumstearat | 0,4 | 0,4 | 0,4 | 0,4 | 0,4 | 0,4 | 0,4 | 0,4 | 0,3 | 0,3 | 0,8 | 0,8 |
| Osmoseschicht | | | | | | | | | | | | |
| HMPC (5 cp) | 4,1 | 4,1 | 4,1 | 4,1 | 4,1 | 4,1 | 4,1 | 4,1 | 3,6 | 3,6 | 8,2 | 8,2 |
| Natriumchlorid | 23,9 | 23,9 | 23,9 | 23,9 | 23,9 | 23,9 | 23,9 | 23,9 | 21,2 | 21,2 | 47,8 | 47,8 |
| PEO 5.000.000 | 52,9 | 52,9 | 52,9 | 52,9 | 52,9 | 52,9 | 52,9 | 52,9 | 47,0 | 47,0 | 105,8 | 105,8 |
| Eisenoxid- rot | 0,8 | 0,8 | 0,8 | 0,8 | 0,8 | 0,8 | 0,8 | 0,8 | 0,7 | 0,7 | 1,6 | 1,6 |
| Magnesiumstearat | 0,2 | 0,2 | 0,2 | 0,2 | 0,2 | 0,2 | 0,2 | 0,2 | 0,2 | 0,2 | 0,4 | 0,4 |
| Osmotische Membran | | | | | | | | | | | | |
| Celluloseacetat | 4,1 | 4,1 | 4,1 | 4,1 | 4,1 | 4,1 | 4,1 | 4,1 | 33,2 | 33,2 | 38,0 | 38,0 |
| PEG 3.350 | 23,9 | 23,9 | 23,9 | 23,9 | 23,9 | 23,9 | 23,9 | 23,9 | 1,7 | 1,7 | 2,0 | 2,0 |
| Wirkstoff-Lack 1* | | | | | | | | | | | | |
| Candesartan cilexetil, mikronisiert | | 8,0 | 16,0 | 16,0 | | 8,0 | 16,0 | 16,0 | 4,0 | 4,0 | 32,0 | 32,0 |
| Opadry II 85F19250 | | 12,0 | 24,0 | 24,0 | | 12,0 | 24,0 | 24,0 | 16,0 | 16,0 | 48,0 | 48,0 |
| Wirkstoff-Lack 2* | | | | | | | | | | | | |
| Hydochlorothiazid, mikronisiert | 12,5 | 12,5 | 12,5 | 25,0 | | | | | 12,5 | | 25,0 | |
| Chlorthalidon, mikronisiert | | | | | 12,5 | 12,5 | 12,5 | 25,0 | | 12,5 | | 50,0 |
| Opadry II 85F19250 | 18,75 | 18,75 | 18,75 | 37,5 | 18,75 | 18,75 | 18,75 | 37,5 | 18,75 | 18,75 | 37,5 | 75,0 |
| Farb-Lack | | | | | | | | | | | | |
| Opadry II 85F230009 | 16,0 | 16,0 | 16,0 | 16,0 | 16,0 | 16,0 | 16,0 | 16,0 | 14,0 | 14,0 | 20,0 | 20,0 |

| | | | | | | | | | | | | |
|---|---|---|---|---|---|---|---|---|---|---|---|---|
| * Die Auftragsreihenfolge der beiden Wirkstoff enthaltenden Lackschichten ist austauschbar. | | | | | | | | | | | | |

### Beispiel 13: Herstellung osmotischer Freisetzungssysteme enthaltend 20 mg Nifedipin mit einer Mantelschicht enthaltend 70 mg Telmisartan

Zur Herstellung der Lacksuspension wurden 264 g mikronisiertes Telmisartan (mit einer Partikelgrößenverteilung beschrieben durch X₅₀ < 2 µm und X₉₀ < 5 µm) in 1584 g gereinigtem Wasser mit einem Propellerrührer suspendiert. Nach Zugabe von 396 g des Fertiglacks Opadry II 85F19250 Clear und weiterem Rühren für ca. 45 Minuten wurde eine homogene Suspension erhalten.

700 g (entsprechend 3240 Stück) Kerne gemäß Beispiel 1c (deklarierter Gehalt = 20 mg Nifedipin) wurden in einen Coater Glatt GC300 eingebracht und bei einer Zulufttemperatur von 60°C, einer Zuluftmenge von 120 m³/h und einer Trommeldrehzahl von 16 UpM (Umdrehungen pro Minute) vorgewärmt. Die Parameter Zulufttemperatur (60°C), Zuluftmenge (120 m³/h) und Trommeldrehzahl (16 UpM) wurden während des gesamten Coating-Prozesses konstant gehalten.

Für das Aufsprühen der Lacksuspension wurde eine Rundstrahldüse mit einem Durchmesser von 0,8 mm bei einem Zerstäuberdruck 1,6 bar verwendet, wobei der bewegliche Sprüharm während des gesamten Coating-Prozesses jeweils so eingestellt war, dass ein homogenes Sprühbild erzielt wurde.

Die Lacksuspension wurde mit einer über die Dauer des Coating-Prozesses stufenweise gesteigerten Sprührate auf die Kerne aufgetragen, und zwar innerhalb der ersten Stunde mit 3,5 g/min und ab der zweiten Stunde mit 7 g/min.

Die Gesamtzeit des Aufsprühens betrug 300 Minuten. Danach wurden die Tabletten für weitere 10 Minuten ohne weiteres Aufsprühen in der Trommel poliert.

Vor dem Coating-Prozess, zu verschiedenen Zeitpunkten während des Coating-Prozesses, und sofort nach dem Coating-Prozess wurden Muster der Tabletten entnommen und die Gewichtszunahme bestimmt. Anhand der Gewichtszunahme wurde der Endpunkt des Aufsprühvorgangs bestimmt.

Die so erhaltenen Tabletten hatten ein Gewicht von 389,5 mg ± 8,1 mg. Dies entspricht einem theoretischen Lackauftrag ca. 173,5 mg. Die erhaltenen Tabletten hatten einen Durchmesser von 9,9 mm und eine Höhe von 5,8 mm. Die erhaltenen Tabletten hatten eine gleichmäßige Oberfläche.

Bei den Inprozesskontrollen wurden folgende Ergebnisse erhalten:

| Zeit [min] | Gewicht [mg] | Standardabweichung [mg] |
|---|---|---|
| 0 | 216,0 | 3,9 |
| 30 | 226,9 | 4,3 |
| 60 | 238,2 | 4.1 |
| 90 | 255,5 | 6,3 |
| 120 | 274,0 | 5,2 |
| 150 | 290,9 | 3,7 |
| 180 | 309,5 | 6,8 |
| 210 | 331,8 | 8,0 |
| 240 | 350,1 | 7,2 |
| 270 | 373,6 | 7,9 |
| 300 | 389,5 | 8,1 |

Die Bruchfestigkeit von 10 der erhaltenen Tabletten wurden mittels eines Bruchfestigkeitstesters (Schleuniger Typ 6D, Dr. Schleuniger Pharmatron AG, Solothurn, Schweiz) untersucht. Die mittlere Bruchfestigkeit betrug 320 N, es wurden Einzelwerte zwischen 280 und 390 N gemessen.

Die erhaltenen Tabletten wurden der Prüfung auf Abrieb gemäß USP 31 (<1216> Tablet Friability) unterzogen. Es konnte kein Abrieb festgestellt werden.

Die erhaltenen Tabletten wurden der Prüfung auf Zerfallszeit gemäß USP 31 (<701> Disintegration) mit Gereinigtem Wasser als Medium bei 37°C unterzogen und fortlaufend beobachtet. Nach spätestens 27 Minuten war der Filmlack vollständig abgelöst.

Die erhaltenen Tabletten wurden einem Freisetzungstest gemäß USP 31 (<711> Dissolution) apparatus 2 (paddle apparatus) mit 75 UpM (Umdrehungen pro Minute) und 900 ml Acetatpuffer pH 4,5 mit einem Zusatz von 0,6 % Natriumlaurylsulfat als Medium bei 37°C unterzogen. Es wurden 6 Tabletten untersucht. Die Wirkstoffgehalte im Freisetzungsmedium wurden mittels HPLC mit UV-Detektion bestimmt. Nach 30 Minuten waren durchschnittlich 65 % des Telmisartans freigesetzt, nach 60 Minuten war das Telmisartan aus allen Tabletten vollständig freigesetzt. Die Freisetzung von Nifedipin beträgt 10% nach 4 Stunden, 55 % nach 12 Stunden und 90 % nach 24 Stunden.

Von 10 zufällig ausgewählten Tabletten den erhaltenen Tabletten wurde jeweils der Einzelgehalt an Telmisartan mittels HPLC mit UV-Detektion bestimmt. Der Mittelwert der Einzelgehalte betrug 70,0 mg bei einer Standardabweichung von 5,7% bezogen auf den Mittelwert. Daraus ergibt sich bei der Prüfung auf Gehaltseinheitlichkeit (content uniformity) ein Akzeptanzwert von 13,7 %.

## Patentansprüche

1. Pharmazeutische Darreichungsform enthaltend eine Wirkstoffkombination von Nifedipin oder Nisoldipin und mindestens einem Angiotensin-II Antagonisten und/oder mindestens einem Diuretikum, **dadurch gekennzeichnet, dass** der Kern ein osmotisches Freisetzungssystem ist, **dadurch gekennzeichnet, dass** die Hülle des osmotischen Freisetzungssystems aus Celluloseacetat oder einem Gemisch von Celluloseacetat und Polyethylenglykol besteht sowie weiterhin **dadurch gekennzeichnet, dass** sich Nifedipin oder Nisoldipin im Kern und der Angiotensin-II Antagonist und/oder das Diuretikum in einem Mantelüberzug um den Kern befinden und **dadurch gekennzeichnet, dass** der Mantelüberzug mindestens ein Film bildendes Polymer geeignet für die schnelle Freisetzung von Wirkstoffen enthält, wobei das Film bildende Polymer partiell hydrolysierter Polyvinylalkohol ist und weiterhin **dadurch gekennzeichnet, dass** mindestens 85 % des Nifedipins oder Nisoldipins (bezogen auf die deklarierte Gesamtmenge des jeweiligen Wirkstoffes) über einen Zeitraum von 24 Stunden, 5 bis 17 % des Nifedipins oder Nisoldipins innerhalb von 4 Stunden und 43 bis 80 % des Nifedipins oder Nisoldipins innerhalb von 12 Stunden freigesetzt werden gemäß USP-Freisetzungsmethode mit Apparatur 2 (Paddle) und mindestens 60 % des Angiotensin-II Antagonisten und/oder des Diuretikums (bezogen auf die deklarierte Gesamtmenge des jeweiligen Wirkstoffes) innerhalb von 30 Minuten freigesetzt werden gemäß USP-Freisetzungsmethode mit Apparatur 2 (Paddle) bei 75 Umdrehungen pro Minute in 900 ml geeignetem Mediums.

2. Pharmazeutische Darreichungsform gemäß Anspruch 1, wobei der Angiotensin-II Antagonist aus der Gruppe Candesartan, Losartan, Telmisartan, Irbesartan, Embursartan, Eprosartan, Valsartan oder Olmesartan ausgewählt ist, wobei Candesartan in Form von Candesartan cilexetil und Olmesartan in Form von Olmesartan medoxomil verwendet wird.

3. Pharmazeutische Darreichungsform gemäß Anspruch 1 wobei der Angiotensin-II Antagonist aus der Gruppe Azilsartan, Candesartan, Losartan, Telmisartan, Irbesartan, Embursartan, Eprosartan, Valsartan oder Olmesartan ausgewählt ist, wobei Candesartan in Form von Candesartan cilexetil und Olmesartan in Form von Olmesartan medoxomil verwendet wird.

4. Pharmazeutische Darreichungsform gemäß einem oder mehreren der Ansprüche 1 bis 3, wobei das Diuretikum aus der Gruppe Acetazolamid, Dichlorphenamid, Methazolamid, Furosemid, Torasemid, Bumetanid, Etacrynsäure, Piretanid, Amilorid, Triamteren, Spironolacton, Kaliumcanreonat, Eplerenon, Hydrochlorothiazid, Chlorthalidon, Xipamid, Metolazon, Mefrusid oder Indapamid ausgewählt ist.

5. Pharmazeutische Darreichungsform gemäß einem oder mehreren der Ansprüche 1 bis 4, **dadurch gekennzeichnet, dass** Nifedipin oder Nisoldipin in einer Mindestdosis von 5 mg und einer Maximaldosis von 90 mg und der Angiotensin-II Antagonist in einer Mindestdosis von 2 mg und einer Maximaldosis von 500 mg und/oder das Diuretikum in einer Mindestdosis von 0,5 mg und einer Maximaldosis von 500 mg eingesetzt werden.

6. Pharmazeutische Darreichungsform gemäß einem oder mehreren der Ansprüche 1 bis 5, **dadurch gekennzeichnet, dass** im Mantelüberzug der Anteil des Angiotensin-II Antagonisten und/oder des Diuretikums 10 bis 50 % bezogen auf das Trockengewicht des Mantelüberzugs beträgt.

7. Pharmazeutische Darreichungsform gemäß einem oder mehreren der Ansprüche 1 bis 6, **dadurch gekennzeichnet, dass** das Gewicht des Mantelüberzugs in der erfindungsgemäßen Darreichungsform 20 bis 300 mg beträgt.

8. Pharmazeutische Darreichungsform gemäß einem oder mehreren der Ansprüche 1 bis 7, **dadurch gekennzeichnet, dass** die Dicke des Mantelüberzugs 50 bis 1500 µm beträgt.

9. Pharmazeutische Darreichungsform gemäß einem oder mehreren der Ansprüche 1 bis 8, **dadurch gekennzeichnet, dass** der Angiotensin II Antagonist Candesartan ist.

10. Pharmazeutische Darreichungsform gemäß einem oder mehreren der Ansprüche 1 bis 8, **dadurch gekennzeichnet, dass** der Angiotensin II Antagonist Candesartan cilexetil ist.

11. Pharmazeutische Darreichungsform gemäß Anspruch 10, **dadurch gekennzeichnet, dass** Nifedipin in einer Menge von 20 mg enthalten ist, und dass Candesartan cilexetil in einer Menge von 4 mg enthalten ist.

12. Pharmazeutische Darreichungsform gemäß Anspruch 10, **dadurch gekennzeichnet, dass** Nifedipin in einer Menge von 20 mg enthalten ist, und dass Candesartan cilexetil in einer Menge von 8 mg enthalten ist.

13. Pharmazeutische Darreichungsform gemäß Anspruch 10, **dadurch gekennzeichnet, dass** Nifedipin in einer Menge von 20 mg enthalten ist, und dass Candesartan cilexetil in einer Menge von 16 mg enthalten ist.

14. Pharmazeutische Darreichungsform gemäß Anspruch 10, **dadurch gekennzeichnet, dass** Nifedipin in einer Menge von 20 mg enthalten ist, und dass Candesartan cilexetil in einer Menge von 32 mg enthalten ist.

15. Pharmazeutische Darreichungsform gemäß Anspruch 10, **dadurch gekennzeichnet, dass** Nifedipin in einer Menge von 30 mg enthalten ist, und dass Candesartan cilexetil in einer Menge von 4 mg enthalten ist.

16. Pharmazeutische Darreichungsform gemäß Anspruch 10, **dadurch gekennzeichnet, dass** Nifedipin in einer Menge von 30 mg enthalten ist, und dass Candesartan cilexetil in einer Menge von 8 mg enthalten ist.

17. Pharmazeutische Darreichungsform gemäß Anspruch 10, **dadurch gekennzeichnet, dass** Nifedipin in einer Menge von 30 mg enthalten ist, und dass Candesartan cilexetil in einer Menge von 16 mg enthalten ist.

18. Pharmazeutische Darreichungsform gemäß Anspruch 10, **dadurch gekennzeichnet, dass** Nifedipin in einer Menge von 30 mg enthalten ist, und dass Candesartan cilexetil in einer Menge von 32 mg enthalten ist.

19. Pharmazeutische Darreichungsform gemäß Anspruch 10, **dadurch gekennzeichnet, dass** Nifedipin in einer Menge von 60 mg enthalten ist, und dass Candesartan cilexetil in einer Menge von 4 mg enthalten ist.

20. Pharmazeutische Darreichungsform gemäß Anspruch 10, **dadurch gekennzeichnet, dass** Nifedipin in einer Menge von 60 mg enthalten ist, und dass Candesartan cilexetil in einer Menge von 8 mg enthalten ist.

21. Pharmazeutische Darreichungsform gemäß Anspruch 10, **dadurch gekennzeichnet, dass** Nifedipin in einer Menge von 60 mg enthalten ist, und dass Candesartan cilexetil in einer Menge von 16 mg enthalten ist.

22. Pharmazeutische Darreichungsform gemäß Anspruch 10, **dadurch gekennzeichnet, dass** Nifedipin in einer Menge von 60 mg enthalten ist, und dass Candesartan cilexetil in einer Menge von 32 mg enthalten ist.

23. Pharmazeutische Darreichungsform gemäß einem oder mehreren der Ansprüche 1 bis 22, **dadurch gekennzeichnet, dass** das osmotische Freisetzungssystem ein osmotisches Einkammersystem ist.

24. Pharmazeutische Darreichungsform gemäß einem oder mehreren der Ansprüche 1 bis 22, **dadurch gekennzeichnet, dass** das osmotische Freisetzungssystem ein osmotisches Zweikammersystem ist.

25. Pharmazeutische Darreichungsform gemäß Anspruch 24, **dadurch gekennzeichnet, dass** das osmotische Freisetzungssystem ein osmotisches Zweikammersystem ist, umfassend
einen Kern mit einer Wirkstoffschicht, enthaltend
• 5 bis 50 % des Wirkstoffes Nifedipin oder Nisoldipin,
• 40 bis 95 % eines oder mehrerer osmotisch aktiver Polymere,
und eine Osmoseschicht, enthaltend
• 40 bis 90 % von einem oder mehreren osmotisch aktiven Polymeren
• 5 bis 40 % eines osmotisch aktiven Zusatzes,
sowie eine Hülle, bestehend aus einem wasserdurchlässigen, für die Komponenten des Kerns undurchlässigen Material mit mindestens einer Öffnung.

26. Verfahren zur Herstellung einer pharmazeutischen Darreichungsform gemäß einem oder mehreren der der Ansprüche 1 bis 25, **dadurch gekennzeichnet, dass** eine wässrige Lacksuspension enthaltend mindestens einen Angiotensin-II Antagonisten und/oder mindestens ein Diuretikum, mindestens ein Film bildendes Polymer und gegebenenfalls weitere Hilfsstoffe auf die Kerne bei einer Zulufttemperatur von 40 bis 70°C aufgesprüht wird.

## Claims

1. Pharmaceutical dosage form comprising an active compound combination of nifedipine or nisoldipine and at least one angiotensin II antagonist and/or at least one diuretic, **characterized in that** the core is an osmotic release system, **characterized in that** the coat of the osmotic release system consists of cellulose acetate or a mixture of cellulose acetate and polyethylene glycol and furthermore **characterized in that** nifedipine or nisoldipine is located in the core and the angiotensin II antagonist and/or the diuretic is located in a mantle coating around the core and **characterized in that** the mantle coating comprises at least one film-forming polymer suitable for the rapid release of active compounds, wherein the film-forming polymer is partially hydrolysed polyvinyl alcohol, and furthermore **characterized in that** at least 85% of the nifedipine or nisoldipine (based on the declared total amount of the respective active compound) are released over a period of 24 hours, 5 to 17% of the nifedipine or nisoldipine are released over 4 hours and 43 to 80% of the nifedipine or nisoldipine are released over 12 hours according to USP release method using apparatus 2 (paddle) and at least 60% of the angiotensin II antagonist and/or the diuretic (based on the declared total amount of the respective active compound) are released over 30 minutes according to USP release method using apparatus 2 (paddle) at 75 revolutions per minute in 900 ml of suitable medium.

2. Pharmaceutical dosage form according to Claim 1, where the angiotensin II antagonist is selected from the group consisting of candesartan, losartan, telmisartan, irbesartan, embursartan, eprosartan, valsartan and olmesartan, where candesartan is used in the form of candesartan cilexetil and olmesartan is used in the form of olmesartan medoxomil.

3. Pharmaceutical dosage form according to Claim 1, where the angiotensin II antagonist is selected from the group consisting of azilsartan, candesartan, losartan, telmisartan, irbesartan, embursartan, eprosartan, valsartan and olmesartan, where candesartan is used in the form of candesartan cilexetil and olmesartan is used in the form of olmesartan medoxomil.

4. Pharmaceutical dosage form according to one or more of Claims 1 to 3, where the diuretic is selected from the group consisting of acetazolamide, dichlorphenamide, methazolamide, furosemide, torasemide, bumetanide, etacrynic acid, piretanide, amiloride, triamterene, spironolactone, potassium canreonate, eplerenone, hydrochlorothiazide, chlorthalidone, xipamide, metolazone, mefruside and indapamide.

5. Pharmaceutical dosage form according to one or more of Claims 1 to 4, **characterized in that** nifedipine or nisoldipine is employed in a minimum dose of 5 mg and a maximum dose of 90 mg and the angiotensin II antagonist in a minimum dose of 2 mg and a maximum dose of 500 mg and/or the diuretic in a minimum dose of 0.5 mg and a maximum dose of 500 mg.

6. Pharmaceutical dosage form according to one or more of Claims 1 to 5, **characterized in that** in the mantle coating the proportion of the angiotensin II antagonist and/or the diuretic is from 10 to 50%, based on the dry weight of the mantle coating.

7. Pharmaceutical dosage form according to one or more of Claims 1 to 6, **characterized in that** the weight of the mantle coating in the dosage form according to the invention is from 20 to 300 mg.

8. Pharmaceutical dosage form according to one or more of Claims 1 to 7, **characterized in that** the thickness of the mantle coating is from 50 to 1500 µm.

9. Pharmaceutical dosage form according to one or more of Claims 1 to 8, **characterized in that** the angiotensin II antagonist is candesartan.

10. Pharmaceutical dosage form according to one or more of Claims 1 to 8, **characterized in that** the angiotensin II antagonist is candesartan cilexetil.

11. Pharmaceutical dosage form according to Claim 10, **characterized in that** it comprises nifedipine in an amount of 20 mg and candesartan cilexetil in an amount of 4 mg.

12. Pharmaceutical dosage form according to Claim 10, **characterized in that** it comprises nifedipine in an amount of 20 mg and candesartan cilexetil in an amount of 8 mg.

13. Pharmaceutical dosage form according to Claim 10, **characterized in that** it comprises nifedipine in an amount of 20 mg and candesartan cilexetil in an amount of 16 mg.

14. Pharmaceutical dosage form according to Claim 10, **characterized in that** it comprises nifedipine in an amount of 20 mg and candesartan cilexetil in an amount of 32 mg.

15. Pharmaceutical dosage form according to Claim 10, **characterized in that** it comprises nifedipine in an amount of 30 mg and candesartan cilexetil in an amount of 4 mg.

16. Pharmaceutical dosage form according to Claim 10, **characterized in that** it comprises nifedipine in an amount of 30 mg and candesartan cilexetil in an amount of 8 mg.

17. Pharmaceutical dosage form according to Claim 10, **characterized in that** it comprises nifedipine in an amount of 30 mg and candesartan cilexetil in an amount of 16 mg.

18. Pharmaceutical dosage form according to Claim 10, **characterized in that** it comprises nifedipine in an amount of 30 mg and candesartan cilexetil in an amount of 32 mg.

19. Pharmaceutical dosage form according to Claim 10, **characterized in that** it comprises nifedipine in an amount of 60 mg and candesartan cilexetil in an amount of 4 mg.

20. Pharmaceutical dosage form according to Claim 10, **characterized in that** it comprises nifedipine in an amount of 60 mg and candesartan cilexetil in an amount of 8 mg.

21. Pharmaceutical dosage form according to Claim 10, **characterized in that** it comprises nifedipine in an amount of 60 mg and candesartan cilexetil in an amount of 16 mg.

22. Pharmaceutical dosage form according to Claim 10, **characterized in that** it comprises nifedipine in an amount of 60 mg and candesartan cilexetil in an amount of 32 mg.

23. Pharmaceutical dosage form according to one or more of Claims 1 to 22, **characterized in that** the osmotic release system is an osmotic one-chamber system.

24. Pharmaceutical dosage form according to one or more of Claims 1 to 22, **characterized in that** the osmotic release system is an osmotic two-chamber system.

25. Pharmaceutical dosage form according to Claim 24, **characterized in that** the osmotic release system is an osmotic two-chamber system comprising
a core with an active compound layer comprising
• from 5 to 50% of the active compound nifedipine or nisoldipine,
• from 40 to 95% of one or more osmotically active polymers,
and an osmosis layer, comprising
• from 40 to 90% of one or more osmotically active polymers
• from 5 to 40% of an osmotically active additive,
and a coat consisting of a water-permeable material which is impermeable for the components of the core and has at least one opening.

26. Process for preparing a pharmaceutical dosage form according to one or more of Claims 1 to 25, **characterized in that** an aqueous coating suspension comprising at least one angiotensin II antagonist and/or at least one diuretic, at least one film-forming polymer and optionally further excipients is sprayed onto the cores at a supply air temperature of from 40 to 70°C.

## Revendications

1. Forme d'administration pharmaceutique contenant une combinaison de substances actives de nifédipine ou de nisoldipine et d'au moins un antagoniste de l'angiotensine-II et/ou au moins un diurétique, **caractérisée en ce que** le noyau est un système de libération osmotique, **caractérisée en ce que** l'enveloppe du système de libération osmotique est constituée d'acétate de cellulose ou d'un mélange d'acétate de cellulose et de polyéthylèneglycol et en outre **caractérisée en ce que** la nifédipine ou la nisoldipine se trouve dans le noyau et l'antagoniste de l'angiotensine-II et/ou le diurétique se trouve dans un revêtement formant une enveloppe autour du noyau et **caractérisée en ce que** le revêtement formant une enveloppe contient au moins un polymère formant un film approprié pour la libération rapide de substances actives, le polymère formant un film étant un poly(alcool vinylique) partiellement hydrolysé et en outre **caractérisée en ce qu'**au moins 85% de la nifédipine ou de la nisoldipine (par rapport à la quantité totale déclarée de chaque substance active) sont libérés sur un laps de temps de 24 heures, 5 à 17% de la nifédipine ou de la nisoldipine sont libérés en 4 heures et 43 à 80% de la nifédipine ou de la nisoldipine sont libérés en 12 heures selon le procédé de libération USP avec un appareil 2 (Paddle) et au moins 60% de l'antagoniste de l'angiotensine-II et/ou du diurétique (par rapport à la quantité totale déclarée de chaque substance active) sont libérés en 30 minutes selon le procédé de libération USP avec un appareil 2 (Paddle) à 75 tours par minute dans 900ml d'un milieu approprié.

2. Forme d'administration pharmaceutique selon la revendication 1, l'antagoniste de l'angiotensine-II étant choisi dans le groupe formé par le candésartan, le losartan, le telmisartan, l'irbésartan, l'embursartan, l'éprosartan, le valsartan ou l'olmésartan, le candésartan étant utilisé sous forme de candésartan cilexetil et l'olmésartan étant utilisé sous forme d'olmésartan médoxomil.

3. Forme d'administration pharmaceutique selon la revendication 1, l'antagoniste de l'angiotensine-II étant choisi dans le groupe formé par l'azilsartan, le candésartan, le losartan, le telmisartan, l'irbésartan, l'embursartan, l'éprosartan, le valsartan ou l'olmésartan, le candésartan étant utilisé sous forme de candésartan cilexetil et l'olmésartan étant utilisé sous forme d'olmésartan médoxomil.

4. Forme d'administration pharmaceutique selon l'une ou plusieurs des revendications 1 à 3, le diurétique étant choisi dans le groupe formé par l'acétazolamide, le dichlorophénamide, le méthazolamide, le furosémide, le torasémide, le bumétanide, l'acide étacrynique, le pirétanide, l'amiloride, le triamtérène, la spironolactone, le canrénoate de potassium, l'éplérénone, l'hydrochlorothiazide, la chlorthalidone, le xipamide, la métolazone, le méfruside ou l'indapamide.

5. Forme d'administration pharmaceutique selon l'une ou plusieurs des revendications 1 à 4, **caractérisée en ce que** la nifédipine ou la nisoldipine sont utilisées en une dose minimale de 5 mg et une dose maximale de 90 mg et l'antagoniste de l'angiotensine-II est utilisé en une dose minimale de 2 mg et une dose maximale de 500 mg et/ou le diurétique est utilisé en une dose minimale de 0,5 mg et une dose maximale de 500 mg.

6. Forme d'administration pharmaceutique selon l'une ou plusieurs des revendications 1 à 5, **caractérisée en ce que** dans le revêtement formant une enveloppe, la proportion de l'antagoniste de l'angiotensine-II et/ou du diurétique est de 10 à 50% par rapport au poids sec du revêtement formant une enveloppe.

7. Forme d'administration pharmaceutique selon l'une ou plusieurs des revendications 1 à 6, **caractérisée en ce que** le poids du revêtement formant une enveloppe dans la forme d'administration selon l'invention est de 20 à 300 mg.

8. Forme d'administration pharmaceutique selon l'une ou plusieurs des revendications 1 à 7, **caractérisée en ce que** l'épaisseur du revêtement formant une enveloppe est de 50 à 1500 µm.

9. Forme d'administration pharmaceutique selon l'une ou plusieurs des revendications 1 à 8, **caractérisée en ce que** l'antagoniste de l'angiotensine-II est le candésartan.

10. Forme d'administration pharmaceutique selon l'une ou plusieurs des revendications 1 à 8, **caractérisée en ce que** l'antagoniste de l'angiotensine-II est le candésartan cilexetil.

11. Forme d'administration pharmaceutique selon la revendication 10, **caractérisée en ce que** la nifédipine est contenue en une quantité de 20 mg et **en ce que** le candésartan cilexetil est contenu en une quantité de 4 mg.

12. Forme d'administration pharmaceutique selon la revendication 10, **caractérisée en ce que** la nifédipine est contenue en une quantité de 20 mg et **en ce que** le candésartan cilexetil est contenu en une quantité de 8 mg.

13. Forme d'administration pharmaceutique selon la revendication 10, **caractérisée en ce que** la nifédipine est contenue en une quantité de 20 mg et **en ce que** le candésartan cilexetil est contenu en une quantité de 16 mg.

14. Forme d'administration pharmaceutique selon la revendication 10, **caractérisée en ce que** la nifédipine est contenue en une quantité de 20 mg et **en ce que** le candésartan cilexetil est contenu en une quantité de 32 mg.

15. Forme d'administration pharmaceutique selon la revendication 10, **caractérisée en ce que** la nifédipine est contenue en une quantité de 30 mg et **en ce que** le candésartan cilexetil est contenu en une quantité de 4 mg.

16. Forme d'administration pharmaceutique selon la revendication 10, **caractérisée en ce que** la nifédipine est contenue en une quantité de 30 mg et **en ce que** le candésartan cilexetil est contenu en une quantité de 8 mg.

17. Forme d'administration pharmaceutique selon la revendication 10, **caractérisée en ce que** la nifédipine est contenue en une quantité de 30 mg et **en ce que** le candésartan cilexetil est contenu en une quantité de 16 mg.

18. Forme d'administration pharmaceutique selon la revendication 10, **caractérisée en ce que** la nifédipine est contenue en une quantité de 30 mg et **en ce que** le candésartan cilexetil est contenu en une quantité de 32 mg.

19. Forme d'administration pharmaceutique selon la revendication 10, **caractérisée en ce que** la nifédipine est contenue en une quantité de 60 mg et **en ce que** le candésartan cilexetil est contenu en une quantité de 4 mg.

20. Forme d'administration pharmaceutique selon la revendication 10, **caractérisée en ce que** la nifédipine est contenue en une quantité de 60 mg et **en ce que** le candésartan cilexetil est contenu en une quantité de 8 mg.

21. Forme d'administration pharmaceutique selon la revendication 10, **caractérisée en ce que** la nifédipine est contenue en une quantité de 60 mg et **en ce que** le candésartan cilexetil est contenu en une quantité de 16 mg.

22. Forme d'administration pharmaceutique selon la revendication 10, **caractérisée en ce que** la nifédipine est contenue en une quantité de 60 mg et **en ce que** le candésartan cilexetil est contenu en une quantité de 32 mg.

23. Forme d'administration pharmaceutique selon l'une ou plusieurs des revendications 1 à 22, **caractérisée en ce que** le système de libération osmotique est un système osmotique à une chambre.

24. Forme d'administration pharmaceutique selon l'une ou plusieurs des revendications 1 à 22, **caractérisée en ce que** le système de libération osmotique est un système osmotique à deux chambres.

25. Forme d'administration pharmaceutique selon la revendication 24, **caractérisée en ce que** le système de libération osmotique est un système osmotique à deux chambres, comprenant
un noyau présentant une couche de substances actives, contenant
- 5 à 50% de la substance active nifédipine ou nisoldipine,
- 40 à 95% d'un ou de plusieurs polymères osmotiquement actifs
et une couche d'osmose, contenant
- 40 à 90% d'un ou de plusieurs polymères osmotiquement actifs
- 5 à 40% d'un additif osmotiquement actif
ainsi qu'une enveloppe, constituée par un matériau perméable à l'eau, imperméable aux constituants du noyau, présentant au moins une ouverture.

26. Procédé pour la préparation d'une forme d'administration pharmaceutique selon l'une ou plusieurs des revendications 1 à 25, **caractérisé en ce qu'**on pulvérise une suspension aqueuse de laque, contenant au moins un antagoniste de l'angiotensine-II et/ou au moins un diurétique, au moins un polymère formant un film et le cas échéant d'autres adjuvants sur les noyaux à une température de l'air alimenté de 40 à 70°C.
